# FASCICULE DE BREVET EUROPEEN

(11) **EP 1 903 881 B1**
(45) Date de publication et mention de la délivrance du brevet: **07.06.2017**
(21) Numéro de dépôt: 06778858.8
(22) Date de dépôt: 11.07.2006
(51) Int. Cl.: A23C 9/123, A23C 9/12, A23B 7/10, A23C 9/13, A23C 11/10, A23L 33/135

(54) **PROCÉDÉ de PRÉPARATION DE PRODUITS ALIMENTAIRES FERMENTES CONTENANT DES SOUCHES PROBIOTIQUES**
VERFAHREN ZUR HERSTELLUNG VON FERMENTIERTEN LEBENSMITTELPRODUKTEN MIT PROBIOTISCHEN STÄMMEN
METHOD FOR PREPARING FERMENTED FOOD PRODUCTS CONTAINING PROBIOTIC STRAINS

(30) Priorité: 13.07.2005 FR 0507529
(43) Date de publication de la demande: 02.04.2008
(73) Titulaire: COMPAGNIE GERVAIS DANONE, 75009 Paris (FR)
(72) Inventeur: TERRAGNO, Luc, F-92190 Meudon (FR); DEBRU, François, F-78000 VERSAILLES (FR); TEiSSIER, Philippe, F-91120 Palaiseau (FR); HERVE, Stéphane, 92330 Sceaux (FR); FAURIE, Jean-Michel, F-78350 Jouy-en-Josas (FR)
(74) Mandataire: Grosset-Fournier, Chantal Catherine
(86) Numéro de dépôt international: PCT/FR2006/001688
(87) Numéro de publication internationale: WO 2007/006970

(56) Documents cités:
- EP-A- 0 281 467
- FR-A- 2 842 707
- JP-A- 2001 321 072
- RU-C1- 2 169 763
- DAVE R I ET AL: "INGREDIENT SUPPLEMENTATION EFFECTS ON VIABILITY OF PROBIOTIC BACTERIA IN YOGHURT" JOURNAL OF DAIRY SCIENCE, AMERICAN DAIRY SCIENCE ASSOCIATION, SAVOY, IL, US, vol. 81, no. 11, novembre 1998 (1998-11), pages 2804-2816, XP000787922 ISSN: 0022-0302
- DECHTER T H ET AL: "SURVIVABILITY AND BETA-GALACTOSIDASE ACTIVITY OF BIFIDOBACTERIA STORED AT LOW TEMPERATURES" FOOD BIOTECHNOLOGY, DEKKER, NEW YORK, NY, US, vol. 12, no. 1/2, 1998, pages 73-89, XP008039285 ISSN: 0890-5436

## Description

L'invention concerne un procédé de préparation des produits alimentaires fermentés contenant des souches probiotiques. Les bifidobactéries font partie de la flore anaérobie dominante du colon. Les principales espèces présentes dans le colon humain sont *Bifidobacterium adolescentis, Bifidobacterium bifidum, Bifidobacterium longum ssp infantis, Bifidobacterium breve, Bifidobacterium longum.*

Les bifidobactéries sont des bactéries probiotiques de choix. Des bactéries du genre *Bifidobacterium* sont utilisées dans de nombreux produits actuellement sur le marché et sont souvent ajoutées à des produits laitiers comprenant déjà les bactéries classiques du yaourt (*Streptococcus thermophilus* et *Lactobacillus bulgaricus*).

La consommation de bifidobactéries est reconnue comme étant bénéfique dans les processus de rétablissement de la population normale de bifidobactéries chez des personnes ayant subi une antibiothérapie. Cette consommation semble aussi permettre de réduire les constipations, de prévenir la survenue de diarrhée et de diminuer les symptômes de l'intolérance au lactose.

Les probiotiques sont des bactéries vivantes. L'utilisation de ces bactéries vivantes dans la fabrication de produits alimentaires tels que des produits laitiers est délicate notamment par rapport aux problématiques de survie de ces bactéries dans le produit.

80% des produits actuellement sur le marché qui contiennent des bifidobactéries ne respectent pas les critères permettant de soutenir qu'ils améliorent de façon significative le transit intestinal des personnes les consommant. Une prise journalière d'au moins 10⁸ à 10⁹ cellules viables a été recommandée comme dose minimale permettant d'avoir un effet thérapeutique (Silva A.M., Barbosa F. H., Duarte R., Vieira L. Q., Arantes R. M., Nicoli J. R., Effect of Bifidobacterium longum ingestion on experimental salmonellosis in mice, J. Appl. Microbiol. 97 (2004) 29-37). La dose requise peut être dépendante de la souche probiotique utilisée.

Dans le cas de la fabrication d'un produit alimentaire bioactif contenant des bifidobactéries se pose donc le problème d'obtenir une population suffisante de ces bactéries dans le produit et de la maintenir au cours de la « vie » du produit, sans recourir à des solutions techniques susceptibles d'altérer les qualités organoleptiques du produit.

Le problème de la taille numérique de la population de souches probiotiques dans un produit laitier fermenté est un problème connu (voir notamment D. Roy, Technological aspects related to the use of bifidobacteria in dairy products, Lait 85 (2005) 39-56, INRA, EDP Sciences).

Plusieurs raisons à ce problème ont été évoquées parmi lesquelles la diminution de la population pendant le stockage, la croissance perturbée de ces bactéries à partir d'un certain pH ou tout simplement la mauvaise capacité de croissance de ces bifidobactéries en particulier dans le lait.

Il est connu que les fructo-oligosaccharides, certains amidons, certains sucres, le glycérol et certains extraits de levures ont des effets bifidogènes importants. *A contrario*, l'oxygène est toxique pour certaines souches probiotiques.

A ce titre, l'utilisation de cystéine ou d'ascorbate en tant que capteur d'oxygène a été décrite (A review of oxygen toxicity in probiotic yogurts : influence on the survival of probiotic bacteria and protective techniques. Talwalkar & Kailasapathy ; Comprehensive Reviews in Food Science and Food Safety, 3 (3) 117-124 ; 2004), sans toutefois qu'il ait été démontré que l'utilisation de ces substances permette d'obtenir et de maintenir au cours du stockage des populations de bifidobactéries aux niveaux souhaités. En outre, l'effet potentiellement négatif de la cystéine sur les propriétés finales d'un yaourt a été relevé. Dave R I et al, Journal of Dairy Science, vol. 81, no. 11, (1998)décrit les effets de l'ajout de cystéine sur la viabilité des bactéries probiotiques. De manière générale, les produits alimentaires fermentés, présentant des propriétés de relatif maintien des populations de bifidobactéries au cours de la conservation desdits produits, et qui sont décrits dans la littérature, ne présentent généralement pas des propriétés organoleptiques acceptables, du fait notamment que des substances telles que de l'extrait de levure sont présentes à une concentration élevée dans les produits. L'invention est définie dans les revendications 1-26. L'invention a principalement pour but de fournir des produits alimentaires fermentés présentant des propriétés organoleptiques acceptables et contenant une forte concentration en bifidobactéries à la fin de la période de fermentation et pendant toute la période de conservation desdits produits alimentaires fermentés.

L'invention a donc aussi pour but de fournir des produits alimentaires fermentés contenant des bifidobactéries en bon état physiologique et présentant un taux de survie important au cours de la période de conservation desdits produits alimentaires fermentés, en particulier jusqu'à la date limite de consommation des produits.

Un autre but de l'invention est de fournir des procédés de préparation simples à mettre en oeuvre permettant d'obtenir les produits ci-dessus.

Un autre but de l'invention est de favoriser la croissance des bifidobactéries par rapport aux symbioses classiques présentes dans les yaourts, ces symbioses étant constituées classiquement d'une ou plusieurs souches de *Streptococcus thermophilus* et de *Lactobacillus bulgaricus.*

Les buts de l'invention sont réalisés grâce à la constatation surprenante effectuée par les inventeurs que l'incorporation d'acides aminés soufrés à la matière de départ lors de la préparation de produits alimentaires fermentés contenant des bifidobactéries, en quantité suffisamment faible pour ne pas altérer les propriétés organoleptiques des produits, permet d'obtenir rapidement après fermentation des populations d'au moins 5.10⁷, voire 10⁸ bifidobactéries par gramme de produit, et une survie accrue des bifidobactéries jusqu'à la date limite de consommation des produits, sans forcément modifier la croissance des autres souches bactériennes.

Par « acide aminé soufré » on entend la cystéine (L-cystéine) ou la méthionine ainsi que leurs dérivés, éventuellement sous forme de sel.

En particulier pourront être utilisés selon l'invention du chlorhydrate monohydraté de L-cystéine (monochlorohydrate monohydraté d'acide (R)-2-amino-mercaptopropionique) ou de la L-méthionine (acide (S)-2-amino-4-méthylthio-butyrique), de formules respectives :

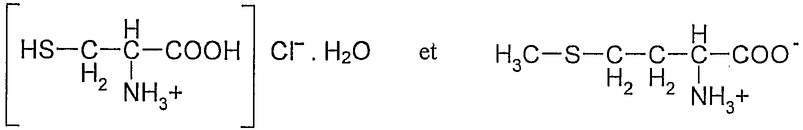

Par « sous forme libre » on entend des acides aminés qui ne sont pas liés à d'autres acides aminés par liaison peptidique au sein de peptides, polypeptides ou protéines.

De préférence, les acides aminés soufrés selon l'invention sont utilisés sous forme réduite, c'est à dire que le groupement sulfhydryle -SH est réduit. Cette forme préférée des acides aminés soufrés exclut donc en particulier la cystine, forme oxydée de la cystéine consistant en l'association de deux cystéines via un pont disulfure.

Les bifidobactéries étant substantiellement dépourvues d'activité protéolytique, il est avantageux d'utiliser les acides aminés susmentionnés sous forme libre afin qu'ils puissent être directement assimilés par les bifidobactéries.

Le ou les acides aminés soufrés utilisés selon l'invention sont avantageusement préalablement filtrés et / ou autoclavés (ou pasteurisés, c'est à dire traités à une température supérieure à 50°C) et / ou irradiés, afin de respecter les contraintes d'usage en matière de contamination microbiologique, c'est à dire afin qu'ils soient substantiellement dépourvus de contaminants microbiens.

Si les acides aminés soufrés sont utilisés à une concentration supérieure à 75 mg/l, une dégradation des propriétés organoleptiques du produit alimentaire est constatée.

Si les acides aminés soufrés sont utilisés à une concentration inférieure à 5 mg/l, la population de bifidobactéries supérieure à 5.10⁷ ou 10⁸ UFC par gramme de produit ne peut généralement pas être maintenue lors de la période de conservation du produit.

Il importe de noter que la concentration d'acides aminés soufrés utilisés selon l'invention concerne le ou les acides aminés soufrés spécialement ajoutés lors de la préparation des produits. Cette concentration ne tient pas compte de l'éventuelle production bactérienne d'acides aminés soufrés lors de la préparation ni même de la quantité d'acides aminés soufrés sous forme libre qui sont naturellement présents dans la matière de départ qui sert à préparer le produit alimentaire (par exemple dans le lait) ou dans les adjuvants qui peuvent intervenir au cours de la préparation.

La concentration typique d'acides aminés soufrés présents dans le lait est de 100 à 1300 mg/l dont environ 260 mg/l pour la cystéine et 1020 mg/l pour la méthionine (Handbook of Milk composition, 1995, Academic Press). Il faut noter que la très grande majorité de ces acides aminés soufrés présents dans le lait est sous forme liée dans des chaînes peptidiques ou protéiques.

Par « ferments » on entend un ensemble de bactéries, notamment des bactéries destinées à la fermentation et / ou des bactéries à valeur probiotique.

Par « propriétés organoleptiques acceptables » on entend notamment l'absence de faux goût de type soufré, telle que déterminée par un test standard d'analyse sensorielle, lequel peut correspondre au protocole décrit ci-après.

Le mécanisme sensoriel débute par la génération d'un stimulus suite à la consommation d'un produit. Ce stimulus permet une perception qui sera dépendante chez l'individu consommateur de facteurs génétiques et physiologiques. Cette perception est ensuite verbalisée (une liste de mots est proposée au consommateur) puis quantifiée (utilisation de gammes). Le consommateur donne alors une estimation globale du produit qu'il a consommé (cette estimation sera influencée par sa culture, ses expériences) et dit s'il serait prêt ou non à acheter ce produit (des données telles que le coût, la communication sur ce produit peuvent être alors apportées).

L'analyse sensorielle est une science basée sur la perception (physiologique et psychologique) impliquant les cinq sens (goût, odorat, vision, audition, toucher) et utilisant des protocoles très rigoureux.

Les consommateurs constituant le panel qui font les analyses sensorielles sont sélectionnés pour leurs capacités sensorielles, leurs capacités en terme de verbalisation, leurs capacités en terme d'utilisation de gammes pour une évaluation et leurs capacités de travail en groupe (pour obtenir des consensus). Il est absolument nécessaire de vérifier que les évaluations des membres du panel seront répétables, reproductibles, avec une homogénéité en terme de discrimination et en terme de classification. Des tests permettant de vérifier ces pré-requis sont répétés plusieurs fois. Le choix des produits se fait selon trois critères principaux : selon l'âge du produit (on choisit les produits d'un même âge), ces produits doivent être représentatifs dans le cas d'une évaluation standard et les produits sont homogènes (peu de différences entre eux). Ces produits sont présentés de manière anonyme et codée, dans un certain ordre et de manière homogène (même température...).

Les conditions environnementales de l'analyse sensorielle sont importantes : il faut bien standardiser l'air conditionné, l'éclairage, l'environnement sonore, la décoration (neutre si possible), l'odeur de la pièce dans laquelle sera effectué l'analyse. Les membres du panel sont par box séparés. Ils ne doivent pas fumer, ni consommer de café, de menthol dans les heures précédant la session d'analyse. Ils ne doivent pas non plus se parfumer et se maquiller.

A l'issue de cette analyse, un produit est à considérer comme à « propriétés organoleptiques acceptables » si les membres du panel n'ont pas détecté de faux goût du type goût soufré dans ce produit.

La période de conservation ou de stockage du produit alimentaire fermenté est la période qui suit immédiatement la fin de processus de préparation du produit alimentaire fermenté et son conditionnement. Lors de cette période de conservation le produit alimentaire fermenté est habituellement conservé à une température comprise entre environ 4 et environ 10 °C.

Le produit alimentaire fermenté susmentionné contient plus de 5.10⁷, en particulier plus de 10⁸ bifidobactéries par gramme de produit alimentaire fermenté en particulier pendant une période de conservation d'au moins 40 jours. Plus particulièrement, le produit alimentaire fermenté susmentionné contient plus de 5.10⁷, en particulier plus de 10⁸ bifidobactéries par gramme de produit alimentaire fermenté jusqu'à la date limite de consommation du produit.

Les dates limites de consommation dépendent des durées légales de conservation fixées par la législation en vigueur, qui peuvent typiquement varier de 15 à 50 jours à partir de la date de fabrication. A titre d'exemple, la durée légale de conservation est généralement de 30 jours pour les produits laitiers frais.

Une population de bifidobactéries qui est supérieure ou égale à 10⁸ UFC/g à la date limite de consommation (D.L.C.) du produit conservé entre 4 et 10°C peut être considérée comme une population suffisante de bifidobactéries compte tenu des recommandations médicales concernant l'apport en bifidobactéries dans l'alimentation.

Par « ne contient pas plus de 0,5 % d'extrait de levure ou d'autolysat de levure », on entend en particulier que le produit alimentaire fermenté susmentionné ne contient pas plus de 0,5 % d'extrait de levure ou d'autolysat de levure à l'issue de son procédé de préparation et / ou que le produit alimentaire fermenté susmentionné ne contient pas plus de 0,5 % d'extrait de levure ou d'autolysat de levure pendant la durée de conservation d'au moins 30 jours, en particulier d'au moins 35 jours, en particulier d'au moins 40 jours ou jusqu'à la date limite de consommation, du produit alimentaire fermenté susmentionné. De plus, le produit alimentaire fermenté susmentionné ne contient pas non plus une quantité supérieure à 0,5 % d'extrait de levure ou d'autolysat de levure lors du procédé de préparation du produit, et notamment au moment de l'inoculation des bactéries et pendant toute la fermentation.

Par « extrait de levure » et « autolysat de levure » on entend des concentrés des composés solubles des cellules de levure. On renvoie à cet égard notamment à l'article « Yeast extracts : production, properties and components » de Rolf Sommer (9^{th} International Symposium on Yeasts), dont sont extraites les informations ci-dessous.

Les extraits de levure sont produits principalement par autolyse, c'est à dire que l'hydrolyse cellulaire est effectuée sans ajout d'autres enzymes. L'extrait de levure ou l'autolysat de levure sont utilisés principalement dans l'industrie de la fermentation et dans l'industrie agro-alimentaire. Le principal matériau de base utilisé pour fabriquer l'extrait de levure est constitué de levures à forte concentration en protéines (souches de *Saccharomyces cerevisiae*) cultivées sur des milieux à base de molasse ou est constitué de levures de bière désamérisées (souches de *Saccharomyces cerevisiae* ou de *Saccharomyces uvarum*). D'autres matériaux de base utilisés sont les levures telles que *Kluyveromyces fragilis* (fermentée sur du lactosérum) ou *Candida utilis* (cultivée sur des déchets riches en glucides issus de l'industrie du bois ou sur de l'éthanol) ou encore des souches spéciales de levures de boulanger, pour produire de l'extrait de levure contenant des nucléotides 5'.

L'autolyse est le procédé de dissociation le plus fréquemment utilisé dans la production d'extrait de levure. Lors de ce procédé, les levures sont dégradées par leurs propres enzymes endogènes. Le procédé d'autolyse peut être initié par un choc osmotique ou de température contrôlé, provoquant la mort cellulaire sans inactiver les enzymes endogènes (en particulier les protéases). Un pH contrôlé, la température et la durée de l'autolyse sont des facteurs décisifs d'un procédé d'autolyse standardisé. En ajoutant des sels ou des enzymes (par exemple des protéases ou des mélanges de protéases et de peptidases) par rapport à l'autolyse « classique », la dégradation protéique des cellules de levures peut être contrôlée.

Outre l'autolyse, l'extrait de levure peut être produit par thermolyse (par exemple en faisant bouillir les levures dans l'eau à 100°C), plasmolyse (traitement avec des solutions fortement salines à une température inférieure à 100°C) et dégradation mécanique (homogénéisation à haute pression ou broyage).

Puis les composés solubles sont séparés des parois cellulaires insolubles et concentrés par évaporateur à agitation ou évaporateur à flot tombant. Suivent ensuite des étapes éventuelles de filtration, de concentration sous vide partiel et de stérilisation rapide. Trois types d'extrait de levure existent : l'extrait de levure liquide (matière sèche : 50 à 65 %) ; l'extrait de levure de type pâte visqueuse (matière sèche : 70 à 80 %) ; et l'extrait de levure en poudre sèche.

Si l'on prend l'exemple d'un extrait de levure en poudre standard utilisé dans l'industrie de la fermentation, la composition est la suivante :

| | |
|---|---|
| Contenu protéique : | 73-75 % |
| Sodium : | moins de 0,5 % |
| Polysaccharides : | moins de 5 % |
| Oligosacharides : | moins de 1 % |
| Lipides : | moins de 0,5 % |

Le contenu protéique se répartit typiquement de la manière suivante :

| | |
|---|---|
| Acides aminés libres : | 35-40 % |
| Di, tri et tétrapeptides (MW < 600 Da) : | 10-15 % |
| Oligopeptides (MW de 2000-3000 Da) : | 40-45 % |
| Oligopeptides (MW de 3000-100000 Da) : | 2-5 % |

La teneur typique en cystéine est de 0,45 %, et la teneur typique en méthionine est de 1,12 % (1,08 % sous forme libre). On préfère l'utilisation d'au moins un acide aminé soufré, à une concentration totale d'environ 5 à environ 30 mg/l, en particulier d'environ 10 à environ 15 mg/l, en particulier d'environ 12 à environ 15 mg/l, et notamment 12,5 mg/l, sous forme libre, pour la mise en oeuvre d'un procédé de préparation d'un produit alimentaire fermenté à l'aide de ferments contenant des bifidobactéries, lequel produit alimentaire fermenté présente des propriétés organoleptiques acceptables, contient plus d'environ 5.10⁷, en particulier plus d'environ 10⁸ bifidobactéries par gramme de produit alimentaire fermenté pendant une période de conservation d'au moins 30 jours, en particulier d'au moins 35 jours et ne contient pas plus de 0,5 % d'extrait de levure ou d'autolysat de levure.

Par ailleurs, on peut préférer un produit alimentaire fermenté, présentant des propriétés organoleptiques acceptables, contenant des ferments comportant plus d'environ 5.10⁷, en particulier plus d'environ 10⁸ bifidobactéries par gramme de produit alimentaire fermenté pendant une période de conservation d'au moins 30 jours, en particulier d'au moins 35 jours et présentant une concentration totale en acides aminés soufrés sous forme libre d'environ 5 à environ 50 mg/l, en particulier d'environ 5 à environ 30 mg/l, en particulier d'environ 5 à environ 20 mg/l, en particulier d'environ 10 à environ 15 mg/l, en particulier d'environ 12 à environ 15 mg/l, et notamment 12,5 mg/l. Plus particulièrement, ledit produit alimentaire fermenté contient des ferments comportant plus d'environ 5.10⁷, en particulier plus d'environ 10⁸ bifidobactéries par gramme de produit alimentaire fermenté pendant une période de conservation d'au moins 40 jours ou jusqu'à la date limite de consommation du produit alimentaire fermenté.

Avantageusement, le produit alimentaire fermenté tel que défini ci-dessus est tel que le rapport entre le nombre de bifidobactéries contenues dans le produit alimentaire fermenté à l'issue de la période de conservation et le nombre de bifidobactéries contenues dans le produit alimentaire fermenté au début de la période de conservation d'au moins 30 jours, en particulier d'au moins 35 jours est d'environ 0,2 à environ 0,8 en particulier d'environ 0,3 à environ 0,7 et en particulier d'environ 0,4 à environ 0,5.

En d'autres termes le taux de survie des bifidobactéries contenues dans le produit alimentaire fermenté entre le début de la période de conservation (c'est à dire la fin du procédé de préparation) et la fin de la période de conservation est compris entre 20 et 80 %, en particulier entre 30 et 70 %, et en particulier entre 40 et 50 %.

Ladite période de conservation est d'au moins 30 jours, en particulier d'au moins 35 jours, mais plus particulièrement d'au moins 40 jours ou s'étend au moins jusqu'à la date limite de consommation du produit alimentaire fermenté. On envisage également un produit alimentaire fermenté conservé pendant une période de conservation d'au moins 30 jours, en particulier d'au moins 35 jours, à une température d'environ 4 à environ 10°C, présentant des propriétés organoleptiques acceptables et contenant des ferments comprenant plus d'environ 5.10⁷, en particulier plus d'environ 10⁸ bifidobactéries par gramme de produit alimentaire fermenté.

Plus particulièrement on peut envisager un produit alimentaire fermenté conservé pendant une période de conservation d'au moins 30 jours, en particulier d'au moins 35 jours, en particulier d'au moins 40 jours, à une température de moins de 12°C ou de moins de 10°C, présentant des propriétés organoleptiques acceptables et contenant des ferments comprenant plus d'environ 5.10⁷, en particulier plus d'environ 10⁸ bifidobactéries par gramme de produit alimentaire fermenté.

De manière préférée, on envisage un produit alimentaire fermenté tel que défini ci-dessus contenant d'environ 5 à environ 50 mg/l, en particulier d'environ 5 à environ 30 mg/l, en particulier d'environ 5 à environ 20 mg/l, en particulier d'environ 10 à environ 15 mg/l, en particulier d'environ 12 à environ 15 mg/l, et notamment 12,5 mg/l, d'acides aminés soufrés et notamment d'environ 5 à environ 50 mg/l, en particulier d'environ 5 à environ 30 mg/l, en particulier d'environ 5 à environ 20 mg/l, en particulier d'environ 10 à environ 15 mg/l, en particulier d'environ 12 à environ 15 mg/l, et notamment 12,5 mg/l de cystéine et / ou d'environ 5 à environ 30 mg/l, en particulier d'environ 5 à environ 15 mg/l, de méthionine.

Afin de doser la cystéine, il est possible d'utiliser un analyseur d'acides aminés tel que le L-8800 High Speed Amino Acid Analyzer (Hitachi High Technologies). Cet analyseur associe une chromatographie d'échange d'ions à une détection colorimétrique à deux longueurs d'onde (570 et 440 nm) après réaction avec la ninhydrine. On peut également recourir à la chromatographie en phase gazeuse couplée à une spectrométrie de masse ou une chromatographie liquide haute performance couplée à une détection fluorimétrique.

L'utilisation plus particulière de cystéine est avantageuse car elle donne lieu expérimentalement à un meilleur effet bifidogène que la méthionine.

L'utilisation plus particulière de méthionine est avantageuse car son coût est moins élevé que celui de l'utilisation de la cystéine.

Avantageusement, ledit produit alimentaire fermenté contient moins d'environ 0,5 % (p/p) de substances contenant plus d'environ 1,7 % d'acides aminés soufrés libres.

Plus particulièrement ledit produit alimentaire fermenté contient moins d'environ 0,5 % (p/p) d'extrait de levure et / ou d'autolysat de levure et / ou d'hydrolysat de protéines de lait, de végétaux, de soja.

La présence éventuelle de substances du type extrait de levure ou autolysat de levure est facilement détectable dans le produit par des méthodes connues. En particulier, les glucanes ou les mannanes apportés par ces substances sont détectables. Par exemple, les glucanes et mannanes étant des fibres, on peut recourir à la méthode du dosage des fibres alimentaires totales, préconisée par l'AFSSA (méthode AOAC 985.29). L'ajout d'extrait de levure ou d'une substance analogue doit également se traduire par une modification complète de la teneur en l'ensemble des 20 acides aminés dans le produit, ainsi que par une modification de la concentration en vitamines et minéraux, par rapport à la composition normale du produit (pour l'exemple du lait, on se réfère notamment au Handbook of milk composition, 1995, Academic Press).

Selon un mode de réalisation préféré, les bifidobactéries contenues dans le produit alimentaire fermenté tel que défini ci-dessus sont du type *Bifidobacterium animalis,* notamment *Bifidobacterium animalis animalis* et / ou *Bidifobacterium animalis lactis,* et / ou *Bifidobacterium breve* et / ou *Bifidobacterium longum* et / ou *Bifidobacterium infantis* et / ou *Bifidobacterium bifidum.*

Avantageusement, le produit alimentaire fermenté tel que défini ci-dessus est à base de jus végétal et notamment de jus de fruit ou de jus de légume tel que du jus de soja, ou de produit laitier et notamment de lait de vache et / ou de lait de chèvre.

Ledit produit alimentaire fermenté peut également être à base de lait de brebis, de lait de chamelle ou de lait de jument.

Par jus végétal on entend un jus réalisé à partir d'extraits végétaux, notamment de soja, de tonyu, d'avoine, de blé, de maïs...

Des exemples de jus de légume sont : le jus de tomate, le jus de betterave, le jus de carotte...

Des exemples de jus de fruit sont : le jus de pomme, d'orange, de fraise, de pêche, d'abricot, de prune, de framboise, de mûre, de groseille, d'ananas, de citron, d'agrume, de pamplemousse, de banane, de kiwi, de poire, de cerise, de fruit de la passion, de mangue, de fruit exotique, le jus multifruit... Selon le procédé de l'invention, les ferments du produit alimentaire fermenté tel que défini ci-dessus contiennent des bactéries du genre *Lactobacillus delbrueckii bulgaricus.* Selon un mode de réalisation préféré, les ferments du produit alimentaire fermenté tel que défini ci-dessus contiennent des bactéries lactiques, en particulier une ou des bactéries du genre *Lactobacillus delbrueckii bulgaricus* et, *Lactobacillus casei* et / ou *Lactobacillus reuteri* et / ou *Lactobacillus acidophilus* et / ou *Lactobacillus helveticus* et / ou *Lactobacillus plantarum,* et / ou des bactéries du type *Lactococcus cremoris* et / ou *Streptococcus thermophilus* et / ou *Lactococcus lactis* et / ou une ou des bactéries du genre *Leuconostoc.*

Avantageusement, le produit alimentaire fermenté tel que défini ci-dessus est tel que la proportion de bifidobactéries dans les ferments est d'environ 20 à environ 80 %, notamment d'environ 30 à environ 70 %, notamment d'environ 40 à environ 60 %, et notamment d'environ 50 %.

Par « proportion de bifidobactéries dans les ferments » on entend la proportion des bifidobactéries par rapport au nombre total de bactéries incluses dans le produit alimentaire fermenté, c'est à dire par rapport à l'ensemble des bifidobactéries et des autres bactéries, notamment les bactéries *Lactococcus, Lactobacillus, Streptococcus...*

Le bon équilibre numérique entre les bifidobactéries et les autres souches bactériennes dans le produit alimentaire fermenté à l'issue du procédé de préparation, et le maintien substantiel de cet équilibre tout au long de la période de conservation, sont des garants essentiels de la qualité du produit alimentaire.

Une proportion de 50 % de bifidobactéries constitue un bon compromis entre les problématiques de coût (les bifidobactéries coûtent cher) et les problématiques d'obtention d'une population correcte de bifidobactéries.

Selon un mode de réalisation préféré, le produit alimentaire fermenté tel que défini ci-dessus se présente sous la forme d'un produit alimentaire fermenté brassé ou d'un produit alimentaire fermenté à boire ou d'un produit alimentaire fermenté ferme ou d'un produit alimentaire fermenté infantile.

Par « produit [...] brassé » on entend un produit, notamment un lait, ensemencé, fermenté, brassé mécaniquement puis conditionné. La fermentation d'un tel produit ne s'effectue pas en pot mais en vrac, dans des cuves. Le caillé est brassé puis refroidi avant d'être conditionné en pots, qui sont stockés au froid. Par caillé on entend un coagulat de protéines notamment de lait.

Par «produit [...] à boire » on entend un produit sous forme substantiellement liquide. Un produit à boire est un produit qui est tel que, après l'étape de brassage mécanique, le produit est battu dans les cuves avant d'être conditionné.

Par « produit [...] ferme » on entend un produit (notamment un lait) ensemencé et directement conditionné dans des pots où il fermente. Après l'ensemencement, le produit est conditionné en pots. Ces pots passent ensuite généralement à l'étuve pendant 3 heures. Les bactéries se reproduisent et consomment le lactose qui est alors transformé partiellement en acide lactique, ce qui modifie la structure des protéines, formant ce que l'on appelle le « gel lactique ». Les pots passent ensuite en chambre froide ventilée ou en tunnel de refroidissement et sont stockés à 2-4°C.

Par «produit [...] infantile» on entend un produit adapté aux besoins du nourrisson, à faible teneur en protéines et en graisse.

Ledit produit alimentaire fermenté peut notamment être un yaourt ou un yoghourt ferme, brassé ou à boire ou une barre contenant de la matière laitière, du kéfir, un biscuit avec un fourrage laitier, une eau contenant des probiotiques... L'invention concerne un procédé de préparation d'un produit laitier alimentaire fermenté à partir d'une matière de départ, comprenant
- une étape d'ensemencement d'une matière de départ, éventuellement pasteurisée, par inoculation de ferments d'ensemencement contenant des des bifidobactéries et des bactéries du genre *Lactobacillus delbrueckii bulgaricus'* pour obtenir une matière ensemencée,
- une étape de fermentation de la matière ensemencée obtenue à l'étape précédente de manière à obtenir une matière fermentée,
- une étape d'incorporation d'au moins un acide aminé soufré sous forme libre à la concentration de 5 à 30 mg/l, en particulier d'environ 5 à environ 20 mg/l, en particulier d'environ 10 à environ 15 mg/l, en particulier d'environ 12 à environ 15 mg/l, et notamment 12,5 mg/l, cette étape d'incorporation pouvant intervenir
   - soit avant l'étape d'ensemencement,
   - soit substantiellement simultanément à l'étape d'ensemencement,
   - soit après l'étape d'ensemencement et avant l'étape de fermentation, sous réserve que le produit alimentaire fermenté ne contienne pas plus de 0,5 % (p/p) d'extrait de levure et / ou d'autolysat de levure.

Par « fermentation » on entend une réaction biochimique qui consiste à libérer de l'énergie à partir d'un substrat organique, sous l'action de micro-organismes. Il s'agit d'un procédé de transformation d'une matière première par les micro-organismes, cette transformation produisant alors de la biomasse et des métabolites. En particulier, la fermentation lactique est un processus anaérobie de consommation du lactose par les bactéries des ferments, ce qui provoque la formation d'acide lactique et un abaissement du pH.

L'invention provient de la constatation surprenante effectuée par les inventeurs que l'incorporation d'acides aminés soufrés dans les gammes sus-citées, en l'absence d'extrait de levure et / ou d'autolysat de levure ou en présence d'une concentration faible de ceux-ci, permet d'améliorer la résistance des bifidobactéries et leur capacité à survivre. Les bidifobactéries contenues dans le produit alimentaire fermenté à l'issue du procédé de préparation de l'invention sont dans un meilleur état physiologique que si l'étape d'incorporation d'acides aminés soufrés était omise, ce qui permet à un plus grand nombre de ces bifidobactéries de survivre au cours de la conservation du produit alimentaire fermenté qui suit.

La cystéine et / ou la méthionine ont donc un effet bifidogène spécifique. En revanche l'utilisation d'extrait de levure et / ou d'autolysat de levure, notamment à des concentrations supérieures à 0,5 % (p/p), a tendance à stimuler l'ensemble des bactéries contenues dans le produit alimentaire fermenté, ce qui peut conduire à un déséquilibre de la symbiose bactérienne en la défaveur des bifidobactéries, et en faveur notamment, si elles sont présentes, des bactéries lactiques. Les conséquences de ce déséquilibre sont une modification du pH, une production d'acide acétique et / ou d'H₂O₂, tous événements préjudiciables à la qualité du produit.

Par ailleurs il faut noter qu'à partir d'une concentration en acides aminés soufrés supérieure à 30 mg/l, en particulier à partir d'une concentration en acides aminés soufrés supérieure à 50 mg/l, et plus particulièrement à partir d'une concentration en acides aminés soufrés supérieures à 75 mg/l, une nette dégradation des propriétés organoleptiques des produits alimentaires est constatée. Cette dégradation est constatée au moyen d'un test de goût standard tel que décrit ci-dessus, qui révèle l'existence d'un goût soufré susceptible de rendre les produits impropres à la consommation et à la commercialisation. Il importe de noter que le goût soufré désagréable survient notamment en cas d'incorporation de cystéine et / ou de méthionine à plus de 75 mg/l, voire dans certains cas à plus de 50 ou 30 mg/l, mais également lorsque les concentrations en acides aminés soufrés dépassent de telles valeurs du fait de la présence de substances supplémentaires, par exemple de l'extrait de levure ou de l'autolysat de levure, notamment à raison de plus de 0,5 % (p/p).

Un autre caractéristique importante du procédé de l'invention est que l'incorporation des ferments contenant les bifidobactéries se fait directement dans la matière de départ destinée à devenir le produit alimentaire fermenté, sans avoir nécessairement recours à des milieux artificiels / synthétiques de croissance intermédiaires.

Selon un mode de réalisation particulier, le procédé tel que défini ci-dessus ne comprend pas d'étape d'ajout de substances supplémentaires contenant un ou des acides aminés soufrés.

Selon un autre mode de réalisation particulier, le procédé tel que défini ci-dessus comprend une étape d'ajout de substances supplémentaires contenant un ou des acides aminés soufrés sous forme libre, la concentration d'acides aminés soufrés sous forme libre dans les substances supplémentaires étant inférieure à environ 1,7 %, de préférence inférieure à environ 0,5 %, et la concentration desdites substances supplémentaires dans le produit alimentaire fermenté étant inférieure à environ 0,5 %.

Plus particulièrement, ladite étape d'ajout de substances supplémentaires peut consister en un ajout d'un extrait de levure et / ou d'un autolysat de levure et / ou d'un hydrolysat de protéines de lait, de végétaux, de soja, à une concentration inférieure à environ 0,5 % (p/p).

De manière préférée, cette étape d'ajout de substances supplémentaires a lieu avant l'étape de fermentation, par exemple substantiellement simultanément à l'étape d'ensemencement et / ou simultanément à l'étape d'incorporation d'au moins un acide aminé soufré.

L'intérêt d'un ajout substantiellement simultanément à l'étape d'ensemencement et / ou simultanément à l'étape d'incorporation d'au moins un acide aminé soufré est d'ordre pratique. Dans ce cas, les substances supplémentaires de type extrait de levure sont au moins partiellement dégradées au cours de la fermentation, car elles servent d'apport nutritionnel aux ferments. Ainsi la concentration des substances supplémentaires de type extrait de levure varie au cours de la fermentation.

Avantageusement, le procédé de préparation d'un produit alimentaire fermenté tel que défini ci-dessus comprend également une étape de pasteurisation ayant lieu avant l'étape d'ensemencement, permettant d'obtenir une matière de départ pasteurisée à partir de la matière de départ.

Par « pasteurisation » on entend la méthode usuelle dans le domaine de la conservation des aliments consistant en un chauffage rapide sans ébullition, suivi d'un refroidissement brusque, permettant la destruction de la plupart des bactéries tout en conservant partiellement les protéines.

Selon un mode de réalisation particulier, l'étape d'incorporation d'au moins un acide aminé soufré a lieu avant l'étape de pasteurisation, le ou les acides aminés soufrés étant incorporés à une concentration d'environ 5 à environ 75 mg/l, en particulier d'environ 5 à environ 30 mg/l, en particulier d'environ 10 à environ 15 mg/l, en particulier d'environ 12 à environ 15 mg/l, et notamment 12,5 mg/l.

L'intérêt d'une incorporation avant l'étape de pasteurisation est d'ordre pratique.

Selon un autre mode de réalisation particulier, l'étape d'incorporation d'au moins un acide aminé soufré a lieu substantiellement simultanément à l'étape d'ensemencement, le ou les acides aminés soufrés étant incorporés à une concentration d'environ 5 à environ 50 mg/l, en particulier d'environ 5 à environ 30 mg/l, en particulier d'environ 5 à environ 20 mg/l, en particulier d'environ 10 à environ 15 mg/l, en particulier d'environ 12 à environ 15 mg/l, et notamment 12,5 mg/l.

L'intérêt d'une incorporation substantiellement simultanément à l'étape d'ensemencement est d'ordre économique (le ou les acides aminés soufrés ne sont pas partiellement détruits par un éventuel traitement thermique ou pasteurisation avant l'ensemencement) et d'ordre pratique.

Selon un autre mode de réalisation particulier, l'étape d'incorporation d'au moins un acide aminé soufré a lieu après l'étape d'ensemencement et avant l'étape de fermentation, le ou les acides aminés soufrés étant incorporés à une concentration d'environ 5 à environ 50 mg/l, en particulier d'environ 5 à environ 30 mg/l, en particulier d'environ 5 à environ 20 mg/l, en particulier d'environ 10 à environ 15 mg/l, en particulier d'environ 12 à environ 15 mg/l, et notamment 12,5 mg/l.

L'intérêt d'une incorporation après l'étape d'ensemencement et avant l'étape de fermentation est d'ordre pratique et assure une survie accrue des bifidobactéries durant le stockage du produit.

Il faut noter que dans le cas où l'incorporation du ou des acides aminés soufrés a lieu avant l'étape de pasteurisation, la quantité d'acides aminés soufrés à incorporer doit être majorée d'environ 30 à 50 % par rapport au cas où cette incorporation a lieu après l'éventuelle étape de pasteurisation, c'est à dire notamment substantiellement simultanément à l'étape d'ensemencement ou après l'étape d'ensemencement. En effet, dans le premier cas une partie des acides aminés soufrés est détruite lors de la pasteurisation.

En d'autres termes, la partie supérieure de la gamme de concentration d'acides aminés soufrés de 50-75 mg/l qui est incluse dans la gamme de concentration d'acides aminés soufrés prévue dans l'invention concerne plus spécifiquement le cas où l'incorporation des acides aminés soufrés a lieu préalablement à une étape de pasteurisation.

Il faut noter qu'il est envisageable de diviser l'étape d'incorporation d'acides aminés soufrés en deux sous-étapes ou plus, qui peuvent éventuellement intervenir à des instants différents dans le procédé selon l'invention. La concentration d'acides aminés soufrés qui est indiquée ci-dessus correspond alors à la concentration totale d'acides aminés soufrés à l'issue des différentes sous-étapes d'incorporation d'acides aminés soufrés.

Selon un mode de réalisation préféré, le procédé de préparation d'un produit alimentaire fermenté tel que défini ci-dessus comprend une étape d'ajout d'une préparation intermédiaire simultanément à l'étape d'ensemencement ou entre l'étape d'ensemencement et l'étape de fermentation, de manière à obtenir, à partir de la matière ensemencée, une matière ensemencée complétée, ou après l'étape de fermentation, de manière à obtenir, à partir de la matière fermentée, une matière fermentée complétée, ladite préparation intermédiaire comprenant une préparation de fruits et / ou de céréales et / ou des additifs tels que des arômes et colorants, et ladite étape d'ajout d'une préparation intermédiaire pouvant avoir lieu simultanément à l'étape d'incorporation d'au moins un acide aminé soufré.

La préparation intermédiaire peut notamment contenir des épaississants (fibres solubles et insolubles, alginates, carraghénanes, gomme xanthane, pectine, amidon, notamment gélatinisé, gomme gélane, cellulose et ses dérivés, gomme de guar et de caroube, inuline) ou des édulcorants (aspartame, acésulfame K, saccharine, sucralose, cyclamate) ou des conservateurs.

Des exemples d'arômes sont : l'arôme pomme, orange, fraise, kiwi, coco...

Des exemples de colorants sont : le beta carotène, le carmin, le rouge de cochenille.

En outre, la préparation de fruits sus-citée peut comprendre des fruits entiers ou en morceaux ou en gelée ou en confiture, permettant par exemple d'obtenir des yaourts aux fruits.

La préparation intermédiaire peut encore contenir des extraits végétaux (soja, riz...).

Selon un autre mode de réalisation de l'invention, l'étape d'ensemencement comprend l'inoculation de ferments d'ensemencement contenant d'environ 10⁶ à environ 2.10⁸, plus particulièrement d'environ 10⁶ à environ 10⁷ bifidobactéries, par ml (ou par gramme) de matière de départ.

Si on inocule une quantité de bifidobactéries supérieure à cette gamme, des faux goûts de type acide acétique sont susceptibles de se développer. Si on inocule une quantité de bifidobactéries inférieure à cette gamme, la quantité finale de bifidobactéries est insuffisante.

Avantageusement, dans le procédé de préparation d'un produit alimentaire fermenté selon l'invention, les bifidobactéries sont choisies parmi les bactéries du type *Bifidobacterium animalis,* notamment *Bifidobacterium animalis animalis* et / ou *Bidifobacterium animalis lactis,* et / ou *Bifidobacterium breve* et / ou *Bifidobacterium longum* et / ou *Bifidobacterium infantis* et / ou *Bifidobacterium bifidum.*

De manière particulièrement préférée, dans le procédé de préparation d'un produit alimentaire fermenté selon l'invention, les bifidobactéries sont choisies parmi les bactéries du type *Bifidobacterium animalis.*

Avantageusement, dans le procédé de préparation d'un produit alimentaire fermenté selon l'invention, les ferments d'ensemencement contiennent des bactéries lactiques, en particulier une ou des bactéries du genre *Lactobacillus spp.* et notamment *Lactobacillus delbrueckii bulgaricus* et / ou *Lactobacillus casei* et / ou *Lactobacillus reuteri* et / ou *Lactobacillus acidophilus* et / ou *Lactobacillus helveticus* et / ou *Lactobacillus plantarum,* et / ou des bactéries du type *Lactococcus cremoris* et / ou *Streptococcus thermophilus* et / ou *Lactococcus lactis* et / ou une ou des bactéries du genre *Leuconostoc.*

Selon un mode de réalisation avantageux du procédé de préparation d'un produit alimentaire fermenté de l'invention, la proportion des bifidobactéries dans les ferments d'ensemencement est d'environ 20 à environ 75 %, notamment d'environ 30 à environ 50 %, notamment d'environ 35 à environ 40 %, et notamment d'environ 37,5 %.

Par « proportion des bifidobactéries dans les ferments d'ensemencement », on entend la proportion des bifidobactéries par rapport à l'ensemble total des bactéries inoculées lors de l'étape d'ensemencement.

Cette proportion correspond à un optimum en terme de coût et de concentration finale de bifidobactéries étant donné que plus la concentration de bifidobactéries est élevée au départ, plus elles emportent la compétition en terme de croissance par rapport aux autres souches des ferments, et plus la concentration optimale de bifidobactéries est atteinte rapidement.

Selon un mode de réalisation préféré du procédé de préparation d'un produit alimentaire fermenté de l'invention, la matière de départ est à base de jus végétal et notamment de jus de fruit ou de jus de légume tel que du jus de soja, ou de produit laitier et notamment de lait de vache et / ou de lait de chèvre.

La matière de départ peut également comprendre du lait de brebis et / ou du lait de chamelle et / ou du lait de jument. Le produit alimentaire fermenté étant un produit laitier, la matière de départ peut comprendre du lait, de la poudre de lait, du sucre, un mélange de lait et de jus végétal, un mélange de lait et de jus de fruit, un mélange de lait et d'amidon.

Avantageusement, le procédé de préparation d'un produit alimentaire fermenté selon l'invention est tel que la matière de départ pasteurisée est une matière de départ pasteurisée, chambrée, facultativement homogénéisée, et refroidie, obtenue à partir d'une matière brute, ledit procédé comprenant avant l'étape d'ensemencement les étapes successives suivantes :
- une étape de standardisation en matière grasse de la matière brute de manière à obtenir une matière standardisée,
- une étape d'enrichissement en matière sèche de la matière standardisée obtenue à l'étape précédente, de manière à obtenir une matière enrichie,
- une étape de pré-chauffage de la matière enrichie obtenue à l'étape précédente, de manière à obtenir une matière de départ,
- une étape de pasteurisation et de chambrage de la matière de départ obtenue à l'étape précédente, de manière à obtenir une matière pasteurisée et chambrée,
- une étape facultative d'homogénéisation de la matière pasteurisée et chambrée obtenue à l'étape précédente, de manière à obtenir une matière pasteurisée, chambrée et facultativement homogénéisée,
- une étape de refroidissement initial de la matière pasteurisée, chambrée et facultativement homogénéisée obtenue à l'étape précédente, de manière à obtenir une matière de départ pasteurisée, chambrée, facultativement homogénéisée, et refroidie.

Par « standardisation en matière grasse » on entend une étape de mise à niveau prédéterminé de la quantité de matière grasse présente dans la matière de départ.

L'enrichissement en matière sèche consiste en l'ajout de protéines et de matière grasse pour modifier la fermeté du caillé.

Le chambrage consiste en une thermisation rapide du lait et permet de détruire la flore microbienne végétative, dont les formes pathogènes. Sa durée typique est de 4 à 10 minutes, notamment de 5 à 8 minutes, et notamment d'environ 6 minutes.

Par «homogénéisation» on entend la dispersion de la matière grasse dans la matière de type lait en petits globules gras. L'homogénéisation s'effectue par exemple à une pression de 100 à 280 bars, notamment de 100 à 250 bars, notamment de 100 à 200 bars, notamment d'environ 200 bars. Cette étape d'homogénéisation est purement facultative. Elle est notamment absente dans le processus de production de produits à 0 % de matière grasse.

Selon un mode de réalisation particulier, le procédé de préparation d'un produit alimentaire fermenté tel que défini ci-dessus comprend une étape de conditionnement entre l'étape d'ensemencement et l'étape de fermentation, ladite étape de conditionnement permettant d'obtenir, à partir de la matière ensemencée obtenue à l'étape d'ensemencement, une matière ensemencée et conditionnée.

Ce mode particulier de réalisation correspond au cas des produits alimentaires fermentés de type ferme.

Plus particulièrement, le procédé de préparation d'un produit alimentaire fermenté tel que défini ci-dessus comprend :
- une étape d'ensemencement d'une matière de départ, éventuellement pasteurisée, par inoculation de ferments d'ensemencement contenant environ 10⁶ à environ 2.10⁸, plus particulièrement d'environ 10⁶ à environ 10⁷ bifidobactéries par ml de matière de départ pour obtenir une matière ensemencée,
- une étape de conditionnement de la matière ensemencée obtenue à l'étape précédente, pour obtenir une matière ensemencée conditionnée,
- une étape de fermentation de la matière ensemencée conditionnée obtenue à l'étape précédente, telle que la température de début de fermentation est d'environ 36 à environ 43°C, en particulier d'environ 37 à environ 40°C, la température de fin de fermentation est d'environ 37 à environ 44°C en en particulier d'environ 38 à environ 41°C, et le temps de fermentation est d'environ 6 à environ 11 heures, pour obtenir une matière fermentée,
- une étape de refroidissement final de la matière fermentée obtenue à l'étape précédente, telle que la température de début de refroidissement final est inférieure à environ 22°C et la température de fin de refroidissement final est d'environ 4 à environ 10°C de manière à obtenir un produit alimentaire fermenté.

Selon un mode alternatif de réalisation, ne concernant pas la préparation de produits de type ferme, le procédé de préparation d'un produit alimentaire fermenté selon l'invention comprend les étapes successives suivantes après l'étape de fermentation :
- une étape de refroidissement intermédiaire de la matière fermentée obtenue à l'étape de fermentation, de manière à obtenir une matière pré-refroidie,
- une étape de stockage de la matière pré-refroidie obtenue à l'étape précédente, de manière à obtenir une matière stockée,
- une étape de refroidissement final de la matière stockée obtenue à l'étape précédente, de manière à obtenir un produit alimentaire fermenté.

Selon un mode de réalisation préféré, ladite étape de fermentation est telle que la température de début de fermentation est d'environ 36 à environ 43°C et en particulier d'environ 37 à environ 40°C, la température de fin de fermentation est d'environ 37 à environ 44°C et en particulier d'environ 38 à environ 41°C, et le temps de fermentation est d'environ 6 à environ 11 heures.

De manière avantageuse, ladite étape de refroidissement intermédiaire est telle que le temps de refroidissement intermédiaire est d'environ 1 heure à environ 4 heures et en particulier d'environ 1h30 à environ 2 heures et la température de refroidissement intermédiaire est d'environ 4 à environ 22°C.

De manière préférée, ladite étape de stockage est telle que le temps de stockage est inférieur ou égal à environ 40 heures.

De manière avantageuse, ladite étape de refroidissement final est telle que la température de début de refroidissement final est inférieure à environ 22°C et la température de fin de refroidissement final est d'environ 4 à environ 10°C.

Selon un mode de réalisation préféré, le procédé de préparation d'un produit alimentaire fermenté selon l'invention comprend :
- une étape d'ensemencement d'une matière de départ, éventuellement pasteurisée, par inoculation de ferments d'ensemencement contenant d'environ 10⁶ à environ 2.10⁸, plus particulièrement d'environ 10⁶ à environ 10⁷ bifidobactéries par ml (ou par gramme) de matière de départ pour obtenir une matière ensemencée,
- une étape de fermentation de la matière ensemencée obtenue à l'étape précédente, telle que la température de début de fermentation est d'environ 36 à environ 43°C, en particulier d'environ 37 à environ 40°C, la température de fin de fermentation est d'environ 37 à environ 44°C en en particulier d'environ 38 à environ 41°C, et le temps de fermentation est d'environ 6 à environ 11 heures, pour obtenir une matière fermentée,
- une étape de refroidissement intermédiaire de la matière fermentée obtenue à l'étape précédente, telle que le temps de refroidissement intermédiaire est d'environ 1 heure à environ 4 heures, en particulier d'environ 1h30 à environ 2 heures et la température de refroidissement intermédiaire est d'environ 4 à environ 22°C, de manière à obtenir une matière pré-refroidie,
- une étape de stockage de la matière pré-refroidie obtenue à l'étape précédente, telle que le temps de stockage est inférieur ou égal à environ 40 heures, de manière à obtenir une matière stockée,
- une étape de refroidissement final de la matière stockée obtenue à l'étape précédente, telle que la température de début de refroidissement final est inférieure à environ 22°C et la température de fin de refroidissement final est d'environ 4 à environ 10°C de manière à obtenir un produit alimentaire fermenté.

Selon un mode de réalisation particulier du procédé de préparation d'un produit alimentaire fermenté tel que défini ci-dessus, une étape supplémentaire de brassage est prévue entre l'étape de fermentation et l'étape de refroidissement intermédiaire, permettant d'obtenir, à partir de la matière fermentée obtenue à l'étape de fermentation, une matière fermentée brassée.

Par « brassage » on entend un procédé d'agitation mécanique à l'aide d'un brasseur à turbine ou à hélice. Il s'agit d'une étape déterminante pour l'onctuosité du produit notamment laitier. Si le brassage est trop violent, il peut se produire une incorporation d'air et une séparation du sérum. Si le brassage est insuffisant, le produit risque de devenir ultérieurement trop épais.

Selon un mode de réalisation particulier, le procédé de préparation d'un produit alimentaire fermenté selon l'invention comprend après l'étape de refroidissement final une étape de conservation du produit alimentaire fermenté à une température comprise entre environ 4 et environ 10°C.

L'invention concerne également un produit alimentaire fermenté tel qu'obtenu à partir du procédé tel que défini ci-dessus.

### Brève description des figures

La **figure 1** représente une comparaison des effets de la cystéine et de la vitamine C sur l'acidification du lait par le ferment de l'exemple 1. En abscisse figure le temps en minutes, en ordonnée le pH. Courbe A : témoin sans vitamine C ni cystéine ; courbe B : vitamine C ; courbe C : cystéine.
La **figure 2** représente l'évolution de la population de bifidobactéries dans la maquette témoin au cours de la conservation à 10°C. En abscisse, le temps de conservation en jours ; en ordonnée, la population de bifidobactéries en UFC/ml. ■ : avec 15 mg/l de cystéine filtrée ; ▲ : sans cystéine.
La **figure 3** représente l'évolution de la population de bifidobactéries dans le lait en fonction du traitement du stimulant. Abscisse : temps de conservation en jour ; ordonnée : population en UFC/ml. Conditions : ■, témoin sans cystéine ni méthionine ; ○, cystéine autoclavée ; ●, cystéine filtrée ; □, méthionine autoclavée ; courbe en pointillés, méthionine filtrée.
La **figure 4** représente le suivi de la population de bifidobactéries dans la maquette produit au cours de la conservation à 10°C. Abscisse : temps de conservation en jours ; ordonnée : population en UFC/ml. ■ : cystéine à 12 mg/l incorporée avant pasteurisation ; ▲ : témoin sans cystéine.

### Exemples

### Exemple 1 : étude du mode d'action de la cystéine en tant que stimulant

On utilise un ferment composé de *Streptococcus thermophilus* (CNCM : I-1630) + *Lactobacillus delbrueckii ssp. bulgaricus* (CNCM : I-1632) + *Lactobacillus delbrueckii ssp. bulgaricus* (CNCM : I-1519) + *Bifidobacterium animalis ssp lactis* (CNCM: I-2494).

Il s'agit dans cet exemple d'étudier le mode d'action de la cystéine en tant que stimulant, et de déterminer si elle présente un effet métabolique ou antioxydant.

La croissance de bifidobactéries dans du lait est mesurée en présence d'une solution de vitamine C (0,5 g/l) réduisant totalement l'oxygène du milieu et d'une solution de cystéine (50 mg/l).

### Constitution de la maquette produit :

Poudre de lait écrémée Milex fournisseur Arla food : 120 g
Eau : quantité suffisante pour 1 kg

On effectue un traitement thermique consistant en une pasteurisation pendant 30 minutes à 95°C en bain-marie bouillonnant

La cystéine provient du fournisseur Sigma. La solution est préparée à 500 g/l, filtrée sur unité filtrante Nalgène 0,2 µm. Cette solution est utilisée injectée stérilement dans la maquette pasteurisée pour une concentration finale de 50 mg/l.

La vitamine C provient du fournisseur Sigma. La solution est préparée à 100 g/l, filtrée sur unité filtrante Nalgène 0,2 µm (cat. 156-4020, Nalge Europe Ltd, Belgique). Cette solution est utilisée injectée stérilement dans la maquette pasteurisée pour une concentration finale de 0,5 g/l.

Les doses d'ensemencement de la maquette produit sont données dans le tableau 1 suivant.

**Tableau 1 : doses d'ensemencement**

| | Volume pour 1 l en µl | | |
|---|---|---|---|
| | Témoin | Cystéine filtrée | Vitamine C |
| I-1630 | 100 | 100 | 100 |
| I-1519 + I-1632 | 220 | 220 | 220 |
| I-2494 | 190 | 190 | 190 |
| Cystéine | | 100 | |
| Vitamine C | | | 5 ml |

L'ensemencement est de 5.10⁶ UFC/ml de Streptococcus thermophilus et de 5.10⁶ UFC/ml de Lactobacillus bulgaricus.

Le suivi de l'acidification de la maquette à 37°C est représenté dans le tableau 2 suivant ainsi que sur la figure 1.

**Tableau 2 : suivi de l'acidification de la maquette**

| | Vitamine C | Cystéine filtrée | Témoin |
|---|---|---|---|
| Ta | 86 | 90 | 83 |
| Vmax | -0,0079 | -0,01046 | -0,00791 |
| pHm | 5,96 | 5,82 | 6,06 |
| Tmax | 184 | 208 | 180 |
| pH0 | 6,5 | 6,6 | 6,7 |
| TpH 5,5 | 260 | 248 | 270 |
| TpH 5 | 391 | 354 | 412 |
| TpH 4,8 | 479 | 417 | 506 |
| J0 UFC/ml | 1,02.10⁸ | 2,79.10⁸ | 1,47.10⁸ |

| | | | |
|---|---|---|---|
| Ta = temps de latence (en minutes) Vmax = vitesse maximale (en unités pH/minute) pHm = pH à la vitesse maximale d'acidification Tmax = temps à Vmax (en minutes) pH0 = pH en début de fermentation TpH 5,5 = temps pour arriver à pH 5,5 (en minutes) TpH 5 = temps pour arriver à pH 5 (en minutes) TpH 4,8 = temps pour arriver à pH 4,8 (en minutes) J0 UFC/ml = quantité de bifidobactéries obtenues à l'issue de la fermentation. | | | |

On constate que la courbe d'acidification en présence de cystéine se distingue de la courbe d'acidification en présence de vitamine C, qui est elle-même quasiment indiscernable de la courbe d'acidification témoin sans vitamine C ni cystéine. Etant donné que la vitamine C est un antioxydant, on en déduit que l'effet stimulant de la cystéine n'est pas un effet antioxydant mais est plus certainement un effet d'apport d'acide aminé essentiel.

### Exemple 2 : détermination de la dose de stimulant cystéine

On utilise un ferment composé de *Streptococcus thermophilus* (CNCM : I-2272) + *Streptococcus thermophilus* (CNCM : I-2773) + *Streptococcus thermophilus* (CNCM : I-2130) + *Lactobacillus delbrueckii ssp. bulgaricus* (CNCM : I-1519) + *Bifidobacterium animalis ssp lactis* (CNCM : I-2494).

Des « maquettes lait » sont constituées par des yoghourts brassés classiques comprenant le ferment décrit ci dessus.

L'utilisation de la cystéine filtrée 0,2 µm a été évaluée dans les « maquettes lait » dans les proportions comprises de 5 mg/l à 50 mg/l (5 à 20 mg/l de manière préférentielle).

Pour la méthode de numération des bifidobactéries, on se référera à M. Grand et al., Quantitative analysis and molecular identification of bifidobacteria strains in probiotic milk products, Eur. Food Res. Technol. 217:90-92 (2003).

La population de bifidobactéries pour l'essai contenant la plus forte concentration de L cystéine est de 3.10⁸ UFC/ml à J+24h, J correspondant à l'instant du conditionnement du produit et reste stable jusqu'à 28 jours de conservation à 10°C. La population du témoin standard (Population témoin J0 : 1.10⁸ UFC/ml) est de 9.10⁷ UFC/ml à 28 jours de conservation à 10°C.

La population de bifidobactéries pour l'essai contenant la plus faible concentration de L cystéine est de 1.10⁸ UFC/ml à J+24h.

Certains produits présentent un faux goût caractérisé par une note de soufre décelable'dès 0,002 % de cystéine ajoutée. En dessous de cette concentration de cystéine les produits sont acceptés : une dose de 0,0015 % représente le bon compromis entre les contraintes organoleptiques et les contraintes en terme de population de *Bifidobacterium* >2.10⁸ UFC/ml.

Des essais de croissance sur lait réalisés en présence de 0,0015 % soit 15 mg/l de cystéine filtrée ont permis d'atteindre une population de Bifidobacterium I-2494 de 2.8.10⁸ UFC/ml après 28 jours de conservation à 10°C (l'évolution de la population par rapport au témoin sans cystéine est représentée sur la figure 2).

D'un point de vue analyse sensorielle, les produits réalisés ne présentent pas de faux goût décelable en comparaison du standard.

### Exemple 3 : cinétique d'acidification

On utilise le ferment décrit dans l'exemple 2.

Les cinétiques d'acidification du lait en présence (15mg/L) de la dose optimale de cystéine et en absence de cystéine (témoin) montrent l'absence d'effet de la cystéine sur la cinétique globale.

### Exemple 4 : Effet du type de traitement de l'acide aminé soufré

On évalue l'impact de la stérilisation par filtration ou thermisation de la cystéine et de la méthionine (concentration finale utilisée : 50 mg/l).

Les solutions sont soit filtrées à 0,2 µm soit autoclavées pendant 5 minutes à 121 °C puis congelées sous forme de billes dans l'azote liquide.

### Constitution de la maquette :

Poudre de lait écrémée Milex fournisseur Arla food : 120 g
Eau : quantité suffisante pour 1 kg
Traitement thermique : pasteurisation 30 minutes à 95°C en bain-marie bouillonnant
Cystéine : fournisseur Sigma. La solution est préparée à 500 g/l, filtrée sur unité filtrante Nalgène 0,2 µm ou stérilisée à 121°C durant 5 minutes par autoclave piloté par sonde de température (Fetinge France S.A., référence KL 60/101). Cette solution est injectée stérilement dans la maquette pasteurisée pour une concentration finale de 50 mg/l.
Méthionine : fournisseur Sigma. La solution est préparée à 300 g/l ; le traitement de filtration ou de stérilisation est identique à celui effectué pour la solution de cystéine. Cette solution est injectée stérilement dans la maquette pasteurisée pour une concentration finale de 50 mg/l.

Les doses d'ensemencement sont répertoriées dans le tableau 3 ci-dessous.

**Tableau 3 : doses d'encemencement**

| Volume pour 1 l en µl | | | | | |
|---|---|---|---|---|---|
| | Témoin | Cystéine filtrée | Cystéine autoclavée | Méthionine filtrée | Méthionine autoclavée |
| I-1630 | 100 | 100 | 100 | 100 | 100 |
| I-1519 + I-1632 | 220 | 220 | 220 | 220 | 220 |
| I-2494 | 95 | 95 | 95 | 95 | 95 |
| Cystéine filtrée | | 100 | | | |
| Cystéine autoclavée | | | 100 | | |
| Méthionine filtrée | | | | 100 | |
| Méthionine autoclavée | | | | | 100 |

L'ensemencement est de 5.10⁶ UFC/ml de Streptococcus thermophilus et de 5.10⁶ UFC/ml de Lactobacillus bulgaricus.

Le suivi de la population de bifidobactéries dans la maquette conservée à 4°C en fonction des diverses conditions ci-dessus est représenté sur la figure 3 ainsi que dans le tableau 4 suivant :

**Tableau 4 : évolution de la population de bifidobactéries**

| | J0 UFC/ml | J10 UFC/ml | J24 UFC/ml | J29 UFC/ml |
|---|---|---|---|---|
| Cystéine filtrée | 5,1.10⁸ | 4,3.10⁸ | 4,0.10⁸ | 2,3.10⁸ |
| Cystéine autoclavée | 3,1.10⁸ | 5,2.10⁸ | 2,8.10⁸ | 1,7.10⁸ |
| Méthionine filtrée | 4,0.10⁸ | 6,2.10⁸ | 3,4.10⁸ | 2,3.10⁸ |
| Méthionine autoclavée | 3,4.10⁸ | 3,5.10⁸ | 3,5.10⁸ | 2,8.10⁸ |
| Témoin | 1,7.10⁸ | 7.10⁷ | 5.10⁷ | 2.10⁷ |

Le temps de référence J0 correspond à la mise en pots (conditionnement). Les mesures à J10, J24, J29 sont effectuées respectivement 10 jours, 24 jours, 29 jours après cette mise en pots.

Dans tous les cas la population de bifidobactéries est augmentée par l'apport de cystéine ou de méthionine. Aucun effet du traitement thermique n'est observable dans les conditions d'essai sur l'efficacité des stimulants. Le traitement thermique appliqué sur la cystéine à 50mg/l n'en dégrade qu'une partie, la concentration résiduelle (non évaluée) est suffisante pour améliorer la population de bifidobactéries.

### Exemple 5 : évolution de la population de bifidobactéries au cours de la conservation en cas d'incorporation de cystéine avant pasteurisation

La dose de 12 mg/l de cystéine est définie comme effet stimulant répondant à la cible population (2.10⁸ UFC/ml) et répondant positivement en terme d'organoleptie (pas de différence détectable). Cette concentration a été évaluée en incorporation directe à la composition maquette et pasteurisée (95°C, 30 min.).

Le suivi de la population de bifidobactéries dans la maquette produit au cours de la conservation à 10°C est représenté en figure 4.

La population de *Bifidobacterium* est de 2,4.10⁸ UFC/ml à J1 (soit 24 h de stockage) et reste stable après 44 jours de conservation à 10°C (au-dessus de 1,4.10⁸ UFC/ml). L'effet stimulant est clairement démontré par rapport au témoin standard (1,6.10⁸ UFC/ml à J1 ; 7,65.10⁷ UFC/ml à J8 ; 2.10⁷ UFC/ml à J28 ; 1,8.10⁷ UFC/ml à J35 ; 8,5.10⁶ UFC/ml à J44) dans ces conditions : la population à J0 est plus élevée quand on utilise les acides aminés soufrés et le maintien de la population au cours de la vie du produit est très amélioré. Cet effet stimulant reste cependant moins efficace que l'ajout de cystéine filtrée 0,2 µm à la maquette (3.10⁸ UFC/ml), le traitement thermique entraînant une dégradation de la cystéine (concentration résiduelle inférieure à 15mg/l). Un surdosage initial de la quantité de cystéine sera à prévoir dans le cas où la cystéine subit un traitement thermique.

Conclusions concernant les conditions de mise en oeuvre de la cystéine :
- la mise en oeuvre de la cystéine en direct filtrée (avec le ferment) préserve la cystéine ;
- son ajout dans la composition de la maquette traitée thermiquement donne un résultat un peu moins bon en terme de population mais il faut tenir compte de la dégradation de la cystéine lors du traitement thermique (moins disponible) ;
- son ajout via un ingrédient laitier (par exemple GlycoMacroPeptide correspondant au fragment 106-169 de la caséine kappa) traité thermiquement donne de moins bons résultats (moins disponible) ;
- son ajout sous forme congelée avec le ferment est possible.

### Exemple 6 : fabrication d'un yoghourt brassé gras selon l'invention à l'échelle du laboratoire (micro-fabrication)

### 1. Composition du lait et réhydratation

Le yoghourt brassé est composé des ingrédients suivants : du lait écrémé à 0% de matières grasses, de la crème à 40% de matières grasses et de la poudre de lait écrémé (PLE) à 33% de protéines.

Dans un premier temps, tous les ingrédients sont associés ensemble afin de standardiser le lait à un taux protéique (TP) de 4,4 %, un taux de matières grasses (MG) de 3,5% et un taux de matières sèches de 15,8% avec agitation du milieu pendant 60 minutes à environ 750 tours/min avec un agitateur HEIDOLPH® afin que les protéines se réhydratent.

Le contrôle de la standardisation est effectué au détecteur infrarouge MILKOSCAN FT 120® de la société FOSS®. Ci-dessous, un exemple des quantités nécessaires de chaque ingrédient pour obtenir les cibles caractérisant le lait.

| Ingrédients | En % |
|---|---|
| Lait écrémé 0% MG | 87,5 |
| Crème à 40% MG | 8,7 |
| Protéines de lait écrémé 33% TP | 3,8 |
| TOTAL | 100 |

### 2. Homogénéisation

Le lait est alors chauffé entre 50°C et 60°C afin de bien fondre les globules de gras. Une fois la température atteinte, les 10 litres sont homogénéisés au MICROFLUIDIZER® de la société MICROCORPS®. Ceci permet de casser les globules gras en passant le lait en capillaires à travers une grille et sous une pression de 350 Bars.

### 3. Pasteurisation

Un bain-marie de la société MEMMERT® est préparé et réglé à 103°C. Le lait est transféré dans 8 bouteilles de 1 litre, avec une pesée précise de cette quantité pour chaque bouteille.

Les bouteilles sont immergées jusqu'au bas du col dans le bain-marie à 103°C pendant 35 minutes, puis 10 minutes à 95°C dans le même bain-marie.

### 4. Refroidissement et stockage

Les bouteilles sont refroidies dans un bain d'eau froide en flux continu, puis stockées à 4°C au réfrigérateur de 12 à 24 heures selon le planning prévu de l'essai.

### 5. Chambrage

Les bouteilles de lait sont sorties du réfrigérateur 45 minutes avant l'inoculation des ferments et placées en bain-marie à la température de fermentation considérée, soit 37°C.

### 6. Fermentation

Après inoculation des ferments (5.10⁶ UFC/ml de Streptococcus thermophilus ; 5.10⁶ UFC/ml de Lactobacillus bulgaricus ; 5.10⁶ UFC/ml de bifidobactéries) et de la L-cystéine (15 mg/l) à la température de fermentation 37°C, les bouteilles sont replongées dans le bain-marie, et l'acidification est suivie par le CINAC® de la société YSEBAERT® jusqu'à un pH de 4,8.

### 7. Décaillage et lissage

Le décaillage de la bouteille se fait à la main. Le yoghourt décaillé est versé dans la trémie de la plate-forme de lissage. Le lissage se fait via une grille métallique de porosité 500 microns et le produit lissé est refroidit à 20°C via un circuit d'échange en eau glacée.

### 8. Conditionnement et stockage

Le conditionnement se fait manuellement en pots de 125 ml et l'opercule est thermoscellé avec la thermoscelleuse DNV-100-25 PPV-A® de la société FESTO®. Les produits sont stockés en chambre froide 10°C pour toute la durée de l'essai.

### Exemple 7 : Evaluation de la dose de cystéine à ajouter pour obtenir un produit à bonne qualité organoleptique et contenant la population cible de Bifidobactéries

Différents produits ont été préparés avec des doses croissantes de cystéine (voir le tableau ci- dessous).

Le témoin était le produit laitier classique contenant le ferment.

**Gamme :**

| volume / 1L | dose Cystéine | |
|---|---|---|
| 3,2 mL | 0,0080% | 80mg/L |
| 2 mL | 0,0050% | 50mg/L |
| 0,8 mL | 0,0020% | 20mg/L |
| 0,4 mL | 0,0010% | 10mg/L |
| 0,2 mL | 0,0005% | 5mg/L |

Chaque produit a été goûté par 4 personnes connaissant très bien le produit de référence d'un point de vue organoleptique. Ces personnes ont donné leur avis en terme de présence de mauvais goûts (goût soufré, note acide), la référence étant le produit classique ne contenant pas de cystéine.

**Résultats**

| Essai | Populations (Ufc/mL) | | | Evaluation sensorielle (n=4) |
|---|---|---|---|---|
| Temps | T0 | T pH4,8 | Tf 495min | |
| 1 | 4,40E+06 | / | 1,70E+08 | Goût soufré et/ou note acide détectée par tous les dégustateurs |
| 0,008% | | | | |
| 2 | 4,70E+06 | 1,90E+08 | 2,40E+08 | Goût soufré et/ou note acide détectée par tous les dégustateurs |
| 0,005% | | | | |
| 3 | 3,30E+06 | 2,50E+08 | 3,10E+08 | Goût soufré et/ou note acide détectée par au moins un dégustateur |
| 0,002% | | | | |
| 4 | / | / | / | Goût soufré et/ou note acide détectée par au moins un dégustateur |
| 0,0015% | | | | |
| 5 | 3,10E+06 | 1,50E+08 | 2,40E+08 | Aucune détection de mauvais goût |
| 0,001% | | | | |
| 6 | 3,90E+06 | 1,10E+08 | 1,20E+08 | Aucune détection de mauvais goût |
| 0,0005% | | | | |

La dose à 0,0015% n'étant pas encore optimale d'un point de vue organoleptique, la dose à 0,00125% a été testée. Cette dose représente un très bon compromis entre la contrainte en terme de maintien de population et la contrainte en terme de qualité organoleptique.

Le profil sensoriel d'un produit additionné de 0,00125% (12,5 mg/l) de cystéine a été réalisé par un jury d'experts entraînés à ce type de dégustation composé de 15 personnes.

Deux répétitions ont été effectuées. Les dégustateurs avaient à juger le produits sur 23 descripteurs. Les résultats sur ces descripteurs (essentiels pour définir la qualité organoleptique du produit par rapport au produit de référence) n'ont pas montré de différence significative néfaste sur ces descripteurs. Ces descripteurs étaient les suivants :
Aspect sur le produit
   - Sérum visuel (appréciation visuelle de la quantité de sérum en surface du produit)
Texture à la cuillère avant brassage
   - Empreinte (relève de la stabilité de la structure du produit) Texture à la cuillère après brassage du produit
   - Epaisseur (résistance au déplacement de la cuillère)
   - Fil (continuité du fil d'écoulement)
   - Couvrant (quantité de produit qui couvre le dos de la cuillère)
Texture en bouche après brassage du produit
   - Fuyant (vitesse de disparition du produit en bouche)
   - Tapissant (tapisse la paroi buccale)
   - Gras (Sensation de gras en bouche)
   - Doux (Sensation tactile de douceur en bouche)
Saveurs
   - Acide
   - Sucrée
   - Amère
   - Astringent
Arômes du lait
   - Goûts déplaisants
   - Crème
   - Beurre
   - Lait
   - Fromage frais
   - Acétaldéhyde
   - Lactosérum
   - Lactone
   - Citron
   - Pomme de terre

Le résultat recherché est une absence de différence significative entre le produit témoin et le produit supplémenté avec de la cystéine.

Dans le cas présent, un produit selon l'invention, supplémenté avec 12,5 mg/l de cystéine ne présente pas de différence significative en terme d'aspect, de texture, de saveurs et de goûts par rapport au produit témoin.

## Revendications

1. Procédé de préparation d'un produit laitier fermenté à partir d'une matière de départ, comprenant
- une étape d'ensemencement d'une matière de départ, éventuellement pasteurisée, par inoculation de ferments d'ensemencement contenant des bifidobactéries et des bactéries du genre *Lactobacillus delbrueckii bulgaricus* pour obtenir une matière ensemencée,
- une étape de fermentation de la matière ensemencée obtenue à l'étape précédente de manière à obtenir une matière fermentée,
- une étape d'incorporation d'au moins un acide aminé soufré sous forme libre à la concentration de 5 à 30 mg/l, en particulier de 5 à 20 mg/l, en particulier de 10 à 15 mg/l, en particulier de 12 à 15 mg/l, et notamment 12,5 mg/l, cette étape d'incorporation pouvant intervenir
- soit avant l'étape d'ensemencement,
- soit substantiellement simultanément à l'étape d'ensemencement,
- soit après l'étape d'ensemencement et avant l'étape de fermentation,
sous réserve que le produit alimentaire fermenté ne contienne pas plus de 0,5 % (p/p) d'extrait de levure et / ou d'autolysat de levure.

2. Procédé de préparation d'un produit laitier fermenté à partir d'une matière de départ selon la revendication 1, comprenant
- une étape d'ensemencement d'une matière de départ, éventuellement pasteurisée, par inoculation de ferments d'ensemencement contenant des bifidobactéries, des bactéries du genre *Lactobacillus delbrueckii bulgaricus* et, des bactéries du genre *Lactobacillus casei* et / ou *Lactobacillus reuteri* et / ou *Lactobacillus acidophilus* et / ou *Laetobacillus helveticus* et / ou *Lactobacillus plantarum,* et / ou *des bactéries du type Lactococcus cremoris* et / ou *Streptococcus thermophilus* et / ou *Lactococcus lactis* et / ou *une ou des bactéries du genre Leuconostoc,* pour obtenir une matière ensemencée,
- une étape de fermentation de la matière ensemencée obtenue à l'étape précédente de manière à obtenir une matière fermentée,
- une étape d'incorporation d'au moins un acide aminé soufré sous forme libre à la concentration de 5 à 30 mg/l, en particulier de 5 à 20 mg/l, en particulier de 10 à 15 mg/l, en particulier de 12 à 15 mg/l, et notamment 12,5 mg/l, cette étape d'incorporation pouvant intervenir
- soit avant l'étape d'ensemencement,
- soit substantiellement simultanément à l'étape d'ensemencement,
- soit après l'étape d'ensemencement et avant l'étape de fermentation,
sous réserve que le produit alimentaire fermenté ne contienne pas plus de 0,5 % (p/p) d'extrait de levure et / ou d'autolysat de levure.

3. Procédé de préparation d'un produit laitier fermenté à partir d'une matière de départ selon l'une des revendications 1 ou 2, ledit produit laitier étant un yaourt fermenté, comprenant
- une étape d'ensemencement d'une matière de départ, éventuellement pasteurisée, par inoculation de ferments d'ensemencement contenant des bifidobactéries, des bactéries du genre *Lactobacillus delbrueckii bulgaricus,* et des bactéries du genre *Streptococcus thermophilus,* pour obtenir une matière ensemencée,
- une étape de fermentation de la matière ensemencée obtenue à l'étape précédente de manière à obtenir une matière fermentée,
- une étape d'incorporation d'au moins un acide aminé soufré sous forme libre à la concentration de 5 à 30 mg/l, en particulier de 5 à 20 mg/l, en particulier de 10 à 15 mg/l, en particulier de 12 à 15 mg/l, et notamment 12,5 mg/l, cette étape d'incorporation pouvant intervenir
- soit avant l'étape d'ensemencement,
- soit substantiellement simultanément à l'étape d'ensemencement,
- soit après l'étape d'ensemencement et avant l'étape de fermentation,
sous réserve que le produit alimentaire fermenté ne contienne pas plus de 0,5 % (p/p) d'extrait de levure et / ou d'autolysat de levure.

4. Procédé selon l'une des revendications 1 à 3, ne comprenant pas d'étape d'ajout de substances supplémentaires contenant un ou des acides aminés soufrés.

5. Procédé selon l'une des revendications 1 3, comprenant une étape d'ajout de substances supplémentaires contenant un ou des acides aminés soufrés sous forme libre, la concentration d'acides aminés soufrés sous forme libre dans les substances supplémentaires étant inférieure à 1,7 %, de préférence inférieure à 0.5 %, et la concentration desdites substances supplémentaires dans le produit alimentaire fermenté étant inférieure à 0,5 %.

6. Procédé selon la revendication 1, 2, 3 ou 5, comprenant une étape d'ajout de substances supplémentaires constituées d'un extrait de levure et / ou d'un autolysat de levure et / ou d'un hydrolysat de protéines de lait, de végétaux, de soja, à une concentration inférieure à 0,5 % (p/p).

7. Procédé de préparation d'un produit laitier fermenté selon l'une des revendications 1 à 6, comprenant également une étape de pasteurisation ayant lieu avant l'étape d'ensemencement, permettant d'obtenir une matière de départ pasteurisée à partir de la matière de départ.

8. Procédé de préparation d'un produit laitier fermenté selon l'une des revendications 1 à 7, dans lequel l'étape d'incorporation d'au moins un acide aminé soufré a lieu substantiellement simultanément à l'étape d'ensemencement.

9. Procédé de préparation d'un produit laitier fermenté selon l'une des revendications 1 à 7, dans lequel l'étape d'incorporation d'au moins un acide aminé soufré a lieu après l'étape d'ensemencement et avant l'étape de fermentation.

10. Procédé de préparation d'un produit laitier fermenté selon l'une des revendications 1 à 9, comprenant une étape d'ajout d'une préparation intermédiaire simultanément à l'étape d'ensemencement ou entre l'étape d'ensemencement et l'étape de fermentation, de manière à obtenir, à partir de la matière ensemencée, une matière ensemencée complétée, ou après l'étape de fermentation, de manière à obtenir, à partir de la matière fermentée, une matière fermentée complétée, ladite préparation intermédiaire comprenant une préparation de fruits et / ou de céréales et / ou des additifs tels que des arômes et colorants, et ladite étape d'ajout d'une préparation intermédiaire pouvant avoir lieu simultanément à l'étape d'incorporation d'au moins un acide aminé soufré.

11. Procédé de préparation d'un produit laitier fermenté selon l'une des revendications 1 à 10, dans lequel l'étape d'ensemencement comprend l'inoculation de ferments d'ensemencement contenant de 10⁶ à 2.10⁸, plus particulièrement de 10⁶ à 10⁷ bifidobactéries par ml de matière de départ.

12. Procédé de préparation d'un produit laitier fermenté selon l'une des revendications 1 à 11, dans lequel les bifidobactéries sont choisies parmi les bactéries du type *Bifidobacterium animalis,* notamment *Bifidobacterium animalis animalis* et / ou *Bidifabacterium animalis lactis,* et / ou *Bifidobacterium breve* et / ou *Bifidobacterium longum* et / ou *Bifidobacterium infantis* et / ou *Bifidobacterium bifidum.*

13. Procédé de préparation d'un produit laitier fermenté selon l'une des revendications 1 à 12, dans lequel les bifidobactéries sont choisies parmi les bactéries du type *Bifidobacterium animalis*.

14. Procédé de préparation d'un produit laitier fermenté selon l'une des revendications 1 à 13, dans lequel la proportion des bifidobactéries dans les ferments d'ensemencement est de 20 à 75 %, notamment de 30 à 50 %, notamment de 35 à 40 %, et notamment de 37,5 %.

15. Procédé de préparation d'un produit laitier fermenté selon l'une des revendications 1 à 14, dans lequel la matière de départ est à base de produit laitier et notamment de lait de vache et / ou de lait de chèvre.

16. Procédé de préparation d'un produit laitier fermenté selon l'une des revendications 1 à 15, dans lequel la matière de départ est une matière de départ pasteurisée, chambrée, facultativement homogénéisée, et refroidie, obtenue à partir d'une matière brute, ledit procédé comprenant avant l'étape d'ensemencement les étapes successives suivantes :
- une étape de standardisation en matière grasse de la matière brute de manière à obtenir une matière standardisée,
- une étape d'enrichissement en matière sèche de la matière standardisée obtenue à l'étape précédente, de manière à obtenir une matière enrichie,
- une étape de pré-chauffage de la matière enrichie obtenue à l'étape précédente, de manière à obtenir une matière de départ,
- une étape de pasteurisation et de chambrage de la matière de départ obtenue à l'étape précédente, de manière à obtenir une matière pasteurisée et chambrée,
- une étape facultative d'homogénéisation de la matière pasteurisée et chambrée obtenue à l'étape précédente, de manière à obtenir une matière pasteurisée, chambrée et facultativement homogénéisée,
- une étape de refroidissement initial de la matière pasteurisée, chambrée et facultativement homogénéisée obtenue à l'étape précédente, de manière à obtenir une matière de départ pasteurisée, chambrée, facultativement homogénéisée, et refroidie.

17. Procédé de préparation d'un produit laitier fermenté selon l'une des revendications 1 à 16, comprenant une étape de conditionnement entre l'étape d'ensemencement et l'étape de fermentation, ladite étape de conditionnement permettant d'obtenir, à partir de la matière ensemencée obtenue à l'étape d'ensemencement, une matière ensemencée et conditionnée.

18. Procédé de préparation d'un produit laitier fermenté selon l'une des revendications 1 à 17, comprenant :
- une étape d'ensemencement d'une matière de départ, éventuellement pasteurisée, par inoculation de ferments d'ensemencement contenant 10⁶ à 2.10⁸, plus particulièrement de 10⁶ à 10⁷ bifidobactéries par ml de matière de départ pour obtenir une matière ensemencée,
- une étape de conditionnement de la matière ensemencée obtenue à l'étape précédente, pour obtenir une matière ensemencée conditionnée,
- une étape de fermentation de la matière ensemencée conditionnée obtenue à l'étape précédente, telle que la température de début de fermentation est de 36 à 43°C, en particulier de 37 à 40°C, la température de fin de fermentation est de 37 à 44°C en en particulier de 38 à 41°C, et le temps de fermentation est de 6 à 11 heures, pour obtenir une matière fermentée,
- une étape de refroidissement final de la matière fermentée obtenue à l'étape précédente, telle que la température de début de refroidissement final est inférieure à 22°C et la température de fin de refroidissement final est de 4 à 10°C de manière à obtenir un produit alimentaire fermenté.

19. Procédé de préparation d'un produit laitier fermenté selon l'une des revendications 1 à 16, comprenant les étapes successives suivantes après l'étape de fermentation :
- une étape de refroidissement intermédiaire, de la matière fermentée obtenue à l'étape de fermentation, de manière à obtenir une matière pré-refroidie,
- une étape de stockage de la matière pré-refroidie obtenue à l'étape précédente, de manière à obtenir une matière stockée,
- une étape de refroidissement final de la matière stockée obtenue à l'étape précédente, de manière à obtenir un produit alimentaire fermenté.

20. Procédé de préparation d'un produit laitier fermenté selon l'une des revendications 1 à 16 ou 19, dans lequel l'étape de fermentation est telle que la température de début de fermentation est de 36 à 43°C et en particulier de 37 à 40°C, la température de fin de fermentation est de 37 à 44°C et en particulier de 38 à 41°C, et le temps de fermentation est de 6 à 11 heures.

21. Procédé de préparation d'un produit laitier fermenté selon l'une des revendications 19 ou 20 dans lequel l'étape de refroidissement intermédiaire est telle que le temps de refroidissement intermédiaire est de 1 heure à 4 heures et en particulier de 1h30 à 2 heures et la température de refroidissement intermédiaire est de 4 à 22°C.

22. Procédé de préparation d'un produit laitier fermenté selon l'une des revendications 19 à 21 dans lequel l'étape de stockage est telle que le temps de stockage est inférieur ou égal à 40 heures.

23. Procédé de préparation d'un produit laitier fermenté selon l'une des revendications 19 à 22, dans lequel l'étape de refroidissement final est telle que la température de début de refroidissement final est inférieure à 22°C et la température de fin de refroidissement final est de 4 à 10°C.

24. Procédé de préparation d'un produit laitier fermenté selon l'une des revendications 1 à 16 ou 19 à 23, comprenant :
- une étape d'ensemencement d'une matière de départ, éventuellement pasteurisée, par inoculation de ferments d'ensemencement contenant 10⁶ à 2.10⁸, plus particulièrement de 10⁶ à 10⁷ bifidobactéries par ml de matière de départ pour obtenir une matière ensemencée,
- une étape de fermentation de la matière ensemencée obtenue à l'étape précédente, telle que la température de début de fermentation est de 36 à 43°C, en particulier de 37 à 40°C, la température de fin de fermentation est de 37 à 44°C en en particulier de 38 à 41°C, et le temps de fermentation est de 6 à 11 heures, pour obtenir une matière fermentée,
- une étape de refroidissement intermédiaire de la matière fermentée obtenue à l'étape précédente, telle que le temps de refroidissement intermédiaire est de 1 heure à 4 heures, en particulier de 1h30 à 2 heures et la température de refroidissement intermédiaire est de 4 à 22°C, de manière à obtenir une matière pré-refroidie,
- une étape de stockage de la matière pré-refroïdie obtenue à l'étape précédente, telle que le temps de stockage est inférieur ou égal à 40 heures, de manière à obtenir une matière stockée,
- une étape de refroidissement final de la matière stockée obtenue à l'étape précédente, telle que la température de début de refroidissement final est inférieure à 22°C et la température de fin de refroidissement final est de 4 à 10°C de manière à obtenir un produit alimentaire fermenté.

25. Procédé de préparation d'un produit laitier fermenté selon l'une des revendications 19 à 24, comprenant une étape supplémentaire de brassage entre l'étape de fermentation et l'étape de refroidissement intermédiaire, permettant d'obtenir, à partir de la matière fermentée obtenue à l'étape de fermentation, une matière fermentée brassée.

26. Procédé de préparation d'un produit laitier fermenté selon l'une des revendications 19 à 25, comprenant après l'étape de refroidissement final une étape de conservation du produit alimentaire fermenté à une température comprise entre 4 et 10°C.

## Patentansprüche

1. Verfahren zur Herstellung eines fermentierten Milchprodukts aus einem Ausgangsmaterial, umfassend:
- einen-Schritt des Aussäens eines, gegebenenfalls pasteurisierten, Augangsmaterials durch Inokulation von Aussaatfermenten, die Bifidobakterien und Bakterien der Gattung Lactobacillus delbrueckii bulgaricus enthalten, um ein ausgesätes Material zu erhalten,
- einen Schritt des Fermentierens des im vorhergehenden Schritt erhaltenen ausgesäten Materials, um ein fermentiertes Material zu erhalten,
- einen Schritt des Einschließens mindestens einer Schwefelaminosäure in freier Form bei einer Konzentration von 5 bis 30 mg/l, vorzugsweise 5 bis 20 mg/l, vorzugsweise 10 bis 15 mg/l, vorzugsweise 12 bis 15 mg/l, und insbesondere 12,5 mg/l, wobei dieser Einschlussschritt erfolgen kann
- entweder vor dem Aussaatschritt,
- oder im Wesentlichen gleichzeitig mit dem Aussaatschritt,
- oder nach dem Aussaatschritt und vor dem Fermentationsschritt,
mit der Maßgabe, dass das fermentierte Lebensmittelprodukt nicht mehr als 0,5 % (w/w) Hefeextrakt und/oder Hefeautolysat enthält.

2. Verfahren zur Herstellung eines fermentierten Milchprodukts aus einem Ausgangsmaterial nach Anspruch 1, umfassend:
- einen Schritt des Aussäens eines, gegebenenfalls pasteurisierten, Augangsmaterials durch Inokulation von Aussaatfermenten, die Bifidobakterien, Bakterien der Gattung Lactobacillus delbrueckii bulgaricus und Bakterien der Gattung Lactobacillus casei und/oder Lactobacillus reuteri und/oder Lactobacillus acidophilus und/oder Lactobacillus helveticus und/oder Lactobacillus plantarum und/oder Bakterien des Typs Lactococcus cremoris und/oder Streptococcus thermophilus und/oder Lactococcus lactis und/oder eine Bakterie oder Bakterien der Gattung Leuconostoc enthalten,
- um ein ausgesätes Material zu erhalten,
- einen Schritt des Fermentierens des im vorhergehenden Schritt erhaltenen ausgesäten Materials, um ein fermentiertes Material zu erhalten,
- einen Schritt des Einschließens mindestens einer Schwefelaminosäure in freier Form bei einer Konzentration von 5 bis 30 mg/l, vorzugsweise 5 bis 20 mg/l, vorzugsweise 10 bis 15 mg/l, vorzugsweise 12 bis 15 mg/l, und insbesondere 12,5 mg/l, wobei dieser Einschlussschritt erfolgen kann
- entweder vor dem Aussaatschritt,
- oder im Wesentlichen gleichzeitig mit dem Aussaatschritt,
- oder nach dem Aussaatschritt und vor dem Fermentationsschritt,
mit der Maßgabe, dass das fermentierte Lebensmittelprodukt nicht mehr als 0,5 % (w/w) Hefeextrakt und/oder Hefeautolysat enthält.

3. Verfahren zur Herstellung eines fermentierten Milchprodukts aus einem Ausgangsmaterial nach einem der Ansprüche 1 oder 2, wobei das Milchprodukt ein fermentiertes Joghurt ist, umfassend:
- einen Schritt des Aussäens eines, gegebenenfalls pasteurisierten, Augangsmaterials durch Inokulation von Aussaatfermenten, die Bifidobakterien, Bakterien der Gattung Lactobacillus delbrueckii bulgaricus und Bakterien der Gattung Streptococcus thermophilus enthalten, um ein ausgesätes Material zu erhalten,
- einen Schritt des Fermentierens des im vorhergehenden Schritt erhaltenen ausgesäten Materials, um ein fermentiertes Material zu erhalten,
- einen Schritt des Einschließens mindestens einer Schwefelaminosäure in freier Form bei einer Konzentration von 5 bis 30 mg/l, vorzugsweise 5 bis 20 mg/l, vorzugsweise 10 bis 15 mg/l, vorzugsweise 12 bis 15 mg/l, und insbesondere 12,5 mg/l, wobei dieser Einschlussschritt erfolgen kann
- entweder vor dem Aussaatschritt,
- oder im Wesentlichen gleichzeitig mit dem Aussaatschritt,
- oder nach dem Aussaatschritt und vor dem Fermentationsschritt,
mit der Maßgabe, dass das fermentierte Lebensmittelprodukt nicht mehr als 0,5 % (w/w) Hefeextrakt und/oder Hefeautolysat enthält.

4. Verfahren nach einem der Ansprüche 1 bis 3, welches den Schritt des Zusetzens von ergänzenden Substanzen, die eine Schwefelaminosäure oder Schwefelaminosäuren enthalten, nicht umfasst.

5. Verfahren nach einem der Ansprüche 1 bis 3, umfassend einen Schritt des Zusetzens von ergänzenden Substanzen, die eine Schwefelaminosäure oder Schwefelaminosäuren in freier Form enthalten, wobei die Konzentration von Schwefelaminosäuren in freier Form in den ergänzenden Substanzen geringer ist als 1,7 %, bevorzugt geringer als 0,5 %, und die Konzentration der ergänzenden Substanzen in dem fermentierten Lebensmittelprodukt geringer ist als 0,5 %.

6. Verfahren nach Anspruch 1, 2, 3 oder 5, umfassend einen Schritt des Zusetzens von ergänzenden Substanzen, die aus einem Hefeextrakt und/oder einem Hefeautolysat und/oder einem Milch-, Pflanzen-, Sojaproteinhydrolysat bei einer Konzentration von weniger als 0,5 % (w/w) bestehen.

7. Verfahren zur Herstellung eines fermentierten Milchprodukts nach einem der Ansprüche 1 bis 6, umfassend auch einen Schritt des Pasteurisierens, der vor dem Aussaatschritt stattfindet, wobei ein pasteurisiertes Ausgangsmaterial aus dem Ausgangsmaterial erhalten werden kann.

8. Verfahren zur Herstellung eines fermentierten Milchprodukts nach einem der Ansprüche 1 bis 7, wobei der Schritt des Einschließens mindestens einer Schwefelaminosäure im Wesentlichen gleichzeitig mit dem Aussaatschritt stattfindet.

9. Verfahren zur Herstellung eines fermentierten Milchprodukts nach einem der Ansprüche 1 bis 8, wobei der Schritt des Einschließens mindestens einer Schwefelaminosäure nach dem Aussaatschritt und vor dem Fermentationsschritt stattfindet.

10. Verfahren zur Herstellung eines fermentierten Milchprodukts nach einem der Ansprüche 1 bis 9, umfassend einen Schritt des Zusetzens einer Zwischenzubereitung gleichzeitig mit dem Aussaatschritt oder zwischen dem Aussaatschritt und dem Fermentationsschritt, um, aus dem ausgesäten Material, ein komplettes ausgesätes Material zu erhalten oder nach dem Fermentationsschritt, um, aus dem fermentierten Material, ein komplettes fermentiertes Material zu erhalten, wobei die Zwischenzubereitung eine Zubereitung von Früchten und/oder Zerealien und/oder Additiven wie Aromen und Farbstoffen umfasst, und wobei der Schritt des Zusetzens einer Zwischenzubereitung gleichzeitig mit dem Schritt des Einschließens mindestens einer Schwefelaminosäure stattfinden kann.

11. Verfahren zur Herstellung eines fermentierten Milchprodukts nach einem der Ansprüche 1 bis 10, wobei der Aussaatschritt die Inokulation von Aussaatfermenten umfasst, die 10⁶ bis 2.10⁸, bevorzugter 10⁶ bis 10⁷, Bifidobakterien pro ml Ausgangsmaterial enthalten.

12. Verfahren zur Herstellung eines fermentierten Milchprodukts nach einem der Ansprüche 1 bis 11, wobei die Bifidobakterien aus Bakterien des Typs Bifidobacterium animalis, insbesondere Bifidobacterium animalis animalis und/oder Bifidobacterium animalis lactis und/oder Bifidobacterium breve und/oder Bifidobacterium longum und/oder Bifidobacterium infantis und/oder Bifidobacterium bifidum ausgewählt werden.

13. Verfahren zur Herstellung eines fermentierten Milchprödukts nach einem der Ansprüche 1 bis 12, wobei die Bifidobakterien aus Bakterien des Typs Bifidobacterium animalis ausgewählt werden.

14. Verfahren, zur Herstellung eines fermentierten Milchprodukts nach einem der Ansprüche 1 bis 13, wobei der Anteil an Bifidobakterien in den Aussaatfermenten 20 bis 75 %, insbesondere 30 bis 50 %, insbesondere 35 bis 40 %, und insbesondere 37,5 %, beträgt.

15. Verfahren zur Herstellung eines fermentierten Milchprodukts nach einem der Ansprüche 1 bis 14, wobei das Ausgangsmaterial auf einem Milchprodukt und insbesondere auf Kuhmilch und/oder auf Ziegenmilch basiert.

16. Verfahren zur Herstellung eines fermentierten Milchprodukts nach einem der Ansprüche 1 bis 15, wobei das Ausgangsmaterial ein pasteurisiertes, temperiertes, gegebenenfalls homogenisiertes, und gekühltes, Ausgangsmaterial ist, das aus einem Rohmaterial erhalten wird, wobei das Verfahren vor dem Aussaatschritt die folgenden aufeinanderfolgenden Schritte umfasst:
- einen Schritt des Standardisierens des Rohmaterials mit Fettmaterial, um ein standardisiertes Material zu erhalten,
- einen Schritt des Anreicherns des im vorhergehenden Schritt erhaltenen standardisierten Materials mit Trockenmaterial, um ein angereichertes Material zu erhalten,
- einen Schritt des Vorwärmens des im vorhergehenden Schritt erhaltenen angereicherten Materials, um ein Ausgangsmaterial zu erhalten,
- einen Schritt des Pasteurisierens und Temperierens des im vorhergehenden Schritt erhaltenen Ausgangsmaterials, um ein pasteurisiertes und temperiertes Material zu erhalten,
- einen, fakultativen Schritt des Homogenisierens des im vorhergehenden Schritt erhaltenen pasteurisierten und temperierten Materials, um ein pasteurisiertes, temperiertes und fakultativ homogenisiertes Material zu erhalten,
- einen Schritt des anfänglichen Abkühlens des im vorhergehenden Schritt erhaltenen pasteurisierten, temperierten und fakultativ homogenisierten Materials, um ein pasteurisiertes, temperiertes, fakultativ homogenisiertes und abgekühltes Material zu erhalten.

17. Verfahren zur Herstellung eines fermentierten Milchprodukts nach einem der Ansprüche 1 bis 16, umfassend einen Schritt des Konditionierens zwischen dem Aussaatschritt und dem Fermentationsschritt, wobei der Konditionierungsschritt gestattet, aus dem im Aussaatschritt erhaltenen ausgesäten Material, ein ausgesätes und konditioniertes Material zu erhalten.

18. Verfahren zur Herstellung eines fermentierten Milchprodukts nach einem der Ansprüche 1 bis 17, umfassend:
- einen Schritt des Aussäens eines, gegebenenfalls pasteurisierten, Augangsmaterials durch Inokulation von Aussaatfermenten, die 10⁶ bis 2.10⁸, bevorzugter 10⁶ bis 10⁷, Bifidobakterien pro ml Ausgangsmaterial enthalten, um ein ausgesätes Material zu erhalten,
- einen Schritt des Konditionierens des im vorhergehenden Schritt erhaltenen ausgesäten Materials, um ein konditioniertes ausgesätes Material zu erhalten,
- einen Schritt des Fermentierens des im vorhergehenden Schritt erhaltenen ausgesäten konditionierten Materials, so dass die Temperatur am Beginn der Fermentation von 36 bis 43°C, vorzugsweise 37 bis 40°C, beträgt, wobei die Temperatur am Ende der Fermentation von 37 bis 44°C, vorzugsweise 38 bis 41°C, beträgt, und die Fermentationszeit 6 bis 11 Stunden beträgt, um ein fermentiertes Material zu erhalten,
- einen Schritt des endgültigen Kühlens des im vorhergehenden Schritt erhaltenen fermentierten Materials, so dass die Temperatur am Beginn der endgültigen Kühlung niedriger ist als 22°C, und die Temperatur am Ende der endgültigen Kühlung 4 bis 10°C beträgt, um ein fermentiertes Lebensmittelprodukt zu erhalten.

19. Verfahren zur Herstellung eines fermentierten Milchprodukts nach einem der Ansprüche 1 bis 16, umfassend die folgenden aufeinanderfolgenden Schritte nach dem Fermentationsschritt:
- einen Schritt des Zwischenabkühlens des im Fermentationsschritt erhaltenen fermentierten Materials, um ein vorabgekühltes Material zu erhalten,
- einen Schritt des Lagerns des im vorhergehenden Schritt erhaltenen vorabgekühlten Materials, um ein gelagertes Material zu erhalten,
- einen Schritt des endgültigen Kühlens des im vorhergehenden Schritt erhaltenen gelagerten Materials, um ein fermentiertes Lebensmittelprodukt zu erhalten.

20. Verfahren zur Herstellung eines fermentierten Milchprodukts nach einem der Ansprüche 1 bis 16 und 19, wobei der Fermentationsschritt derart ist, dass die Temperatur am Beginn der Fermentation 36 bis 43°C und vorzugsweise 37 bis 40°C beträgt, wobei die Temperatur am Ende der Fermentation 37 bis 44°C und vorzugsweise 38 bis 41°C beträgt, und wobei die Fermentationszeit 6 bis 11 Stunden beträgt.

21. Verfahren zur Herstellung eines fermentierten Milchprodukts nach einem der Ansprüche 19 oder 20, wobei der Zwischenabkühlungsschritt derart ist, dass die Zeit der Zwischenabkühlung 1 Stunde bis 4 Stunden und vorzugsweise 1 h 30 bis 2 Stunden beträgt und die Temperatur der Zwischenabkühlung 4 bis 22°C beträgt.

22. Verfahren zur Herstellung eines fermentierten Milchprodukts nach einem der Ansprüche 19 bis 21, wobei der Lagerungsschritt derart ist, dass die Zeit der Lagerung kleiner oder gleich 40 Stunden ist.

23. Verfahren zur Herstellung eines fermentierten Milchprodukts nach einem der Ansprüche 19 bis 22, wobei der Schritt des endgültigen Kühlens derart ist, dass die Temperatur am Beginn der endgültigen Kühlung niedriger ist als 22°C und die Temperatur am Ende der endgültigen Kühlung 4 bis 10°C beträgt.

24. Verfahren zur Herstellung eines fermentierten Milchprodukts nach einem der Ansprüche 1 bis 16 oder 19 bis 23, umfassend:
- einen Schritt des Aussäens eines, gegebenenfalls pasteurisierten, Augangsmaterials durch Inokulation von Aussaatfermenten, die 10⁶ bis 2.10⁸, bevorzugter 10⁶ bis 10⁷, Bifidobakterien pro ml Ausgangsmaterial enthalten, um ein ausgesätes Material zu erhalten,
- einen Schritt des Fermentierens des im vorhergehenden Schritt erhaltenen ausgesäten Materials, so dass die Temperatur am Beginn der Fermentation von 36 bis 43°C, vorzugsweise 37 bis 40°C, beträgt, und die Temperatur am Ende der Fermentation von 37 bis 44°C und vorzugsweise 38 bis 41°C beträgt, und die Zeit der Fermentation 6 bis 11 Stunden beträgt, um ein fermentiertes Material zu erhalten,
- einen Schritt des Zwischenabkühlens des im vorhergehenden Schritt erhaltenen fermentierten Materials, so dass die Zeit der Zwischenabkühlung 1 Stunde bis 4 Stunden, vorzugsweise 1 h 30 bis 2 Stunden, beträgt, und die Temperatur der Zwischenabkühlung 4 bis 22°C beträgt, um ein vorabgekühltes Material zu erhalten,
- einen Schritt des Lagerns des im vorhergehenden Schritt erhaltenen vorabgekühlten Materials, so dass die Zeit der Lagerung kleiner oder gleich 40 Stunden ist, um ein gelagertes Material zu erhalten,
- einen Schritt des endgültigen Kühlens des im vorhergehenden Schritt erhaltenen gelagerten Materials, so dass die Temperatur am Beginn der endgültigen Kühlung niedriger ist als 22°C, und die Temperatur am Ende der endgültigen Kühlung 4 bis 10°C beträgt, um ein fermentiertes Lebensmittelprodukt zu erhalten.

25. Verfahren zur Herstellung eines fermentierten Milchprodukts nach einem der Ansprüche 19 bis 24, umfassend einen ergänzenden Schritt des Brauens zwischen dem Fermentationsschritt und dem Zwischenabkühlungsschritt, wobei, aus dem im Fermentationsschritt erhaltenen fermentierten Material, ein gebrautes fermentiertes Material erhalten werden kann.

26. Verfahren zur Herstellung eines fermentierten Milchprodukts nach einem der Ansprüche 19 bis 25, umfassend nach dem Schritt des endgültigen Kühlens einen Schritt des Konservierens des fermentierten Lebensmittelprodukts bei einer Temperatur zwischen 4 und 10°C.

## Claims

1. Process for the preparation of a fermented milk product from a starting substance, comprising
- a stage of seeding a starting substance, optionally pasteurized, by inoculation with seeding ferments containing bifidobacteria and bacteria of the genus *Lactobacillus delbrueckii bulgaricus,* in order to obtain a seeded substance,
- a stage of fermentation of the seeded substance obtained in the preceding stage in order to obtain a fermented substance,
- a stage of incorporation of at least one sulphur-containing amino acid in the free form at a concentration from 5 to 30 mg/l, in particular from 5 to 20 mg/l, in particular from 10 to 15 mg/l, in particular from 12 to 15 mg/l, and in particular 12.5 mg/l, this stage of incorporation being able to occur
- either before the seeding stage,
- or substantially simultaneously with the seeding stage,
- or after the seeding stage and before the fermentation stage,
providing that the fermented food product does not contain more than 0.5% (w/w) of yeast extract and / or yeast autolysate.

2. Process for the preparation of a fermented milk product from a starting substance according to claim 1, comprising
- a stage of seeding a starting substance, optionally pasteurized, by inoculation with seeding ferments containing bifidobacteria and bacteria of the genus *Lactobacillus delbrueckii bulgaricus* and bacteria of the genus *Lactobacillus casei* and / or *Lactobacillus reuteri* and / or *Lactobacillus acidophilus* and / or *Lactobacillus helveticus* and / or *Lactobacillus plantarum,* and / or bacteria of the type *Lactococcus cremoris* and / or *Streptococcus thermophilus* and / or *Lactococcus lactis* and / or one or more bacteria of the genus *Leuconostoc,* in order to obtain a seeded substance,
- a stage of fermentation of the seeded substance obtained in the preceding stage in order to obtain a fermented substance,
- a stage of incorporation of at least one sulphur-containing amino acid in the free form at a concentration from 5 to 30 mg/l, in particular from 5 to 20 mg/l, in particular from 10 to 15 mg/l, in particular from 12 to 15 mg/l, and in particular 12.5 mg/l, this stage of incorporation being able to occur
- either before the seeding stage,
- or substantially simultaneously with the seeding stage,
- or after the seeding stage and before the fermentation stage,
providing that the fermented food product does not contain more than 0.5% (w/w) of yeast extract and / or yeast autolysate.

3. Process for the preparation of a fermented milk product from a starting substance according to anyone of claims 1 or 2, said milk product being a fermented yogurt comprising
- a stage of seeding a starting substance, optionally pasteurized, by inoculation with seeding ferments containing bifidobacteria, bacteria of the genus *Lactobacillus delbrueckii bulgaricus* and bacteria of the genus *Streptococcus thermophilus,* in order to obtain a seeded substance,
- a stage of fermentation of the seeded substance obtained in the preceding stage in order to obtain a fermented substance,
- a stage of incorporation of at least one sulphur-containing amino acid in the free form at a concentration from 5 to 30 mg/l, in particular from 5 to 20 mg/l, in particular from 10 to 15 mg/l, in particular from 12 to 15 mg/l, and in particular 12.5 mg/l, this stage of incorporation being able to occur
- either before the seeding stage,
- or substantially simultaneously with the seeding stage,
- or after the seeding stage and before the fermentation stage,
providing that the fermented food product does not contain more than 0.5% (w/w) of yeast extract and / or yeast autolysate.

4. Process according to anyone of claims 1 to 3, not comprising a stage of addition of additional substances containing one or more sulphur-containing amino acids.

5. Process according to anyone of claims 1 to 3, comprising a stage of addition of additional substances containing one or more sulphur-containing amino acids in the free form, the concentration of sulphur-containing amino acids in the free form in the additional substances being less than 1.7%, preferably less than 0.5%, and the concentration of said additional substances in the fermented food product being less than approximately 0.5%.

6. Process according to claims 1, 2, 3 or 5, comprising a stage of addition of additional substances constituted by a yeast extract and / or yeast autolysate and / or milk, plant or soy protein hydrolysate at a concentration of less than 0.5% (w/w).

7. Process for the preparation of a fermented milk product according to one of claims 1 to 6, also comprising a pasteurization stage taking place before the seeding stage, making it possible to obtain a pasteurized starting substance from the starting substance.

8. Process for the preparation of a fermented milk product according to one of claims 1 to 7, in which the stage of incorporation of at least one sulphur-containing amino acid takes place substantially simultaneously to the seeding stage.

9. Process for the preparation of a fermented milk product according to one of claims 1 to 7, in which the stage of incorporation of at least one sulphur-containing amino acid takes place after the seeding stage and before the fermentation stage.

10. Process for the preparation of a fermented milk product according to one of claims 1 to 9, comprising a stage of addition of an intermediate preparation simultaneously with the seeding stage or between the seeding stage and the fermentation stage, so as to obtain, from the seeded substance, a completed seeded substance, or after the fermentation stage, so as to obtain, from the fermented substance, a completed fermented substance, said intermediate preparation comprising a preparation of fruits and / or cereals and / or additives such as flavourings and colourings, and said stage of addition of an intermediate preparation can take place simultaneously with the stage of incorporation of at least one sulphur-containing amino acid.

11. Process for the preparation of a fermented milk product according to one of claims 1 to 10, in which the seeding stage comprises inoculation with seeding ferments containing from 10⁶ to 2.10⁸, more particularly from 10⁶ to approximately 10⁷ bifidobacteria, per ml of starting substance.

12. Process for the preparation of a fermented milk product according to one of claims 1 to 11, in which the bifidobacteria are chosen from bacteria of the type *Bifidobacterium animalis,* in particular *Bifidobacterium animalis animalis* and / or *Bidifobacterium animalis lactis,* and / or *Bifidobacterium breve* and / or *Bifidobacterium longum* and / or *Bifidobacterium infantis* and / or *Bifidobacterium bifidum.*

13. Process for the preparation of a fermented milk product according to one of claims 1 to 12, in which the bifidobacteria are chosen from bacteria of the type *Bifidobacterium animalis.*

14. Process for the preparation of a fermented milk product according to one of claims 1 to 13, in which the proportion of bifidobacteria in the seeding ferments is from 20 to 75%, in particular from 30 to 50%, in particular from 35 to 40%, and in particular 37.5%.

15. Process for the preparation of a fermented milk product according to one of claims 1 to 14, in which the starting substance is based on a dairy product, and in particular cow's milk and / or goat's milk.

16. Process for the preparation of a fermented milk product according to one of claims 1 to 15, in which the starting substance is a pasteurized starting substance, which is held, optionally homogenized, and cooled down, obtained from a raw material, said process comprising, before the seeding stage, the following successive stages:
- a stage of standardization of fatty substances of the raw material so as to obtain a standardized substance,
- a stage of enrichment with dried matter of the standardized substance obtained in the preceding stage, so as to obtain an enriched substance,
- a stage of pre-heating of the enriched substance obtained in the preceding stage, so as to obtain a starting substance,
- a stage of pasteurization and holding of the starting substance obtained in the preceding stage, so as to obtain a pasteurized and held substance,
- an optional stage of homogenization of the pasteurized and held substance obtained in the preceding stage, so as to obtain a pasteurized, held and optionally homogenized substance,
- a stage of initial cooling of the pasteurized, held and optionally homogenized substance obtained in the preceding stage, so as to obtain a pasteurized starting substance, held, optionally homogenized, and cooled.

17. Process for the preparation of a fermented milk product according to one of claims 1 to 16, comprising a packaging stage between the seeding stage and the fermentation stage, said packaging stage making it possible to obtain, from the seeded substance obtained in the seeding stage, a seeded and packaged substance.

18. Process for the preparation of a fermented milk product according to one of claims 1 to 17, comprising:
- a stage of seeding a starting substance, optionally pasteurized, by inoculation with seeding ferments containing from 10⁶ to 2.10⁸ bifidobacteria, more particularly from 10⁶ to 10⁷ bifidobacteria per ml of starting substance, in order to obtain a seeded substance,
- a stage of packaging the seeded substance obtained in the preceding stage, in order to obtain a packaged seeded substance,
- a stage of fermentation of the packaged seeded substance obtained in the preceding stage, such that the temperature at the start of fermentation is from 36 to 43°C, in particular from 37 to 40°C, the temperature at the end of fermentation is from 37 to 44°C, in particular from 38 to 41°C, and the fermentation time is from 6 to 11 hours, in order to obtain a fermented substance,
- a stage of final cooling of the fermented substance obtained in the preceding stage, such that the temperature at the start of the final cooling is less than 22°C and the temperature at the end of the final cooling is from 4 to 10°C, so as to obtain a fermented food product.

19. Process for the preparation of a fermented milk product according to one of claims 1 to 16, comprising the following successive stages after the fermentation stage:
- a stage of intermediate cooling of the fermented substance obtained in the fermentation stage, so as to obtain a pre-cooled substance,
- a stage of storage of the pre-cooled substance obtained in the preceding stage, so as to obtain a stored substance,
- a stage of final cooling of the stored substance obtained in the preceding stage, so as to obtain a fermented food product.

20. Process for the preparation of a fermented milk product according to one of claims 1 to 16 or 19, in which the fermentation stage is such that the temperature at the start of fermentation is from 36 to 43°C and in particular from 37 to 40°C, the temperature at the end of fermentation is from 37 to 44°C and in particular from 38 to 41°C, and the fermentation time is from 6 to 11 hours.

21. Process for the preparation of a fermented milk product according to one of claims 19 or 20 in which the intermediate cooling stage is such that the intermediate cooling time is from 1 hour to 4 hours and in particular from 1 hour 30 minutes to 2 hours and the intermediate cooling temperature is from 4 to 22°C.

22. Process for the preparation of a fermented milk product according to one of claims 19 to 21 in which the storage stage is such that the storage time is less than or equal to 40 hours.

23. Process for the preparation of a fermented milk product according to one of claims 19 to 22, in which the final cooling stage is such that the temperature at the start of final cooling is less than 22°C and the temperature at the end of final cooling is from 4 to 10°C.

24. Process for the preparation of a fermented milk product according to one of claims 1 to 16 or 19 to 23, comprising:
- a stage of seeding a starting substance, optionally pasteurized, by inoculation with seeding ferments containing from 10⁶ to 2.10⁸, more particularly from 10⁶ to 10⁷ bifidobacteria per ml of starting substance in order to obtain a seeded substance,
- a stage of fermentation of the seeded substance obtained in the preceding stage, such that the temperature at the start of fermentation is from 36 to 43°C, in particular from 37 to 40°C, the temperature at the end of fermentation is 37 to 44°C, in particular from 38 to 41°C, and the fermentation time is from 6 to 11 hours, in order to obtain a fermented substance,
- a stage of intermediate cooling of the fermented substance obtained in the preceding stage, such that the intermediate cooling time is from 1 hour to 4 hours, in particular from 1 hour 30 minutes to 2 hours and the intermediate cooling temperature is from 4 to 22°C, so as to obtain a pre-cooled substance,
- a stage of storage of the pre-cooled substance obtained in the preceding stage, such that the storage time is less than or equal to 40 hours, so as to obtain a stored substance,
- a stage of final cooling of the stored substance obtained in the preceding stage, such that the temperature at the start of final cooling is less than 22°C and the temperature at the end of final cooling is from 4 to 10°C so as to obtain a fermented food product.

25. Process for the preparation of a fermented milk product according to one of claims 19 to 24, comprising an additional stirring stage between the fermentation stage and the intermediate cooling stage, making it possible to obtain, from the fermented substance obtained in the fermentation stage, a stirred fermented substance.

26. Process for the preparation of a fermented milk product according to one of claims 19 to 25, comprising after the final cooling stage, a stage of preservation of the fermented food product at a temperature comprised between 4 and 10 °C.
